# EUROPEAN PATENT SPECIFICATION

(11) **EP 4 605 548 B1**
(45) Date of publication and mention of the grant of the patent: **25.11.2026**
(21) Application number: 23800757.9
(22) Date of filing: 20.10.2023
(51) Int. Cl.: C12Q 1/6809, C12N 15/10

(54) **METHODS AND PRODUCTS FOR BIOMARKER IDENTIFICATION**
VERFAHREN UND PRODUKTE ZUR BIOMARKERIDENTIFIZIERUNG
PROCÉDÉS ET PRODUITS D'IDENTIFICATION DE BIOMARQUEURS

(30) Priority: 21.10.2022 GB 202215579
(43) Date of publication of application: 27.08.2025
(73) Proprietor: WOBBLE GENOMICS LIMITED, Edinburgh EH4 2HS (GB)
(72) Inventor: KUO, Richard Izen, Edinburgh EH4 2HS (GB)
(74) Representative: Boult Wade Tennant LLP
(86) International application number: PCT/EP2023/079325
(87) International publication number: WO 2024/084059

(56) References cited:
- WO-A1-2014/071029
- WO-A1-2022/229128
- HU YUEMING ET AL: "Improving the diversity of captured full-length isoforms using a normalized single-molecule RNA-sequencing method", COMMUNICATIONS BIOLOGY, vol. 3, no. 1, 30 July 2020 (2020-07-30), XP093105681, Retrieved from the Internet <URL:https://www.nature.com/articles/s42003-020-01125-7.pdf> DOI: 10.1038/s42003-020-01125-7
- BRIAN B. TUCH ET AL: "Tumor Transcriptome Sequencing Reveals Allelic Expression Imbalances Associated with Copy Number Alterations", PLOS ONE, vol. 5, no. 2, 19 February 2010 (2010-02-19), pages e9317, XP055214138, DOI: 10.1371/journal.pone.0009317

## Description

### FIELD OF THE INVENTION

The invention relates to methods, compositions, systems and kits for discovering disease biomarkers and diagnosing diseases. The invention also relates to methods for processing blood samples for use in biomarker discovery and detection.

### BACKGROUND

Screening for diseases can come in many forms, and recently there has been increasing interest in molecular screening approaches. The detection of RNA that is specific to cancer in a simple blood test, for example, has the potential to provide a readily acceptable and relatively non-invasive screening modality, as well as a prognostic biomarker (Larson, M.

H. *et al.* A comprehensive characterization of the cell-free transcriptome reveals tissue- and subtype-specific biomarkers for cancer detection. 1-11). This approach is also becoming affordable, allowing for regular screening intervals, aiding early diagnosis. However, the handling and processing of samples for RNA sequencing is a challenge in the clinical setting. Additionally, developing a method that is sufficiently sensitive and specific has proven to be a challenge. As a result, there are currently no practical and precise blood RNA detection platforms in use clinically for breast or ovarian cancer diagnoses.

With respect to the blood handling, the primary concern is maintaining the integrity of the RNA. RNA is well known for degrading rapidly. In addition, blood and other tissue types contain enzymes which actively degrade RNA. This results in a rapid decline of both the quantity and quality of RNA in blood post collection. Thus, through the process of collecting, storing, and transporting blood, the RNA content is constantly degrading.

RNA expression is characterized by a wide range of expression levels across unique genes. There are a set of highly expressed genes known as housekeeping genes. These genes do not provide useful information, but they typically make up more than half of the total quantity of RNA in any given sample. In blood, this problem is even more extreme as globin RNA and ribosomal RNA typically represent over 95% of the total quantity of RNA (Harrington, C. A. et al. RNA-Seq of human whole blood: Evaluation of globin RNA depletion on Ribo-Zero library method. Sci. Rep. 10, 1-12 (2020)). Thus, a significant challenge with current detection methods is that they either rely on depletion of these genes or the targeting of a subset of genes. These solutions result in a lack of breadth of detection for the overall transcriptome of blood and may explain why this approach has not been successful.

A further problem is how representative the data is of the actual complexity and biological variations. While there are several RNA detection assays, until recently these methods only allowed for the detection of small fragments of each RNA molecule. This is problematic because each gene can be transcribed into a multitude of different RNA isoforms. This is possible due to the multiple combinations of transcription start sites, termination sites, and alternative splicing (Harrow, J. et al. GENCODE : producing a reference annotation for ENCODE. 7, 1-9 (2006). These alternative transcripts often have distinct functions and are often associated with specific cell and tissue types. As a result, the detection of small fragments of RNA alone does not tell the whole story.

Hu et al. (2020) Communications Biology. 3, 403 describes transcriptome profiling of human gastric signet-ring cell carcinomas.

WO 2014/071029 A1 describes gene expression profiling to differentiate progressive from non-progressive T1 bladder cancer.

Tuch et al. (2010) PLOS ONE. 5(2): e9317 relates to tumour transcriptome sequencing.

### SUMMARY OF THE INVENTION

The present inventors have developed technologies that work with long read RNA sequencing in a way that can provide better consistency, sensitivity, and specificity for RNA-based disease screening. RNA or cDNA samples are typically dominated by sequences from highly expressed genes which can negatively affect analysis of the samples. The present invention provides methods and devices for preparing processed nucleic acid samples with a more uniform distribution of sequences, which can then be analysed to discover and/or detect disease biomarkers. Normalization increases the efficiency of sequencing for transcript discovery and/or detection. First, genes and isoforms which are specific to the condition in question are easier to detect, and second, there is less redundancy in data generated reducing data storage requirements. Also described herein are methods designed to protect the RNA within blood samples. The methods may be combined to further improve the ability to discover and/or detect disease biomarkers.

Aspects of the present invention are defined in the independent claims. Preferable features of the invention are defined in the dependent claims.

It should be borne in mind that the various aspects have been devised so as to be advantageously combined and all such combinations are envisaged within the scope of the invention. It should also be appreciated that options described in relation to one area of improvement will apply *mutatis mutandis* to other areas; e.g. sample types, diseases etc. as appropriate.

Described herein is a method for discovering a disease biomarker comprising:
(i) providing a first cDNA sample and a second cDNA sample;
(ii) normalizing the first and the second cDNA samples;
(iii) sequencing the normalized first and second cDNA samples; and
(iv) comparing the sequencing output for the first and second cDNA samples to discover a disease biomarker.

The first cDNA sample and the second cDNA sample may be from the same subject. In certain examples the first cDNA sample is from the subject prior to treatment for a disease and the second cDNA sample is from the same subject after treatment for the disease. In specific examples the first cDNA sample is from the subject prior to treatment for a disease and the second cDNA sample is from the same subject after treatment for the disease has started and/or after treatment for the disease has been completed.

The first cDNA sample and the second cDNA sample may be from different subjects. In specific examples the first cDNA sample and the second cDNA sample are from subjects with the same disease at different grades or stages. In certain examples the first cDNA sample and the second cDNA sample are from subjects with different diseases, for example different types of cancer. In certain examples the first cDNA sample is from a subject with a disease and the second cDNA sample is from a subject without the disease.

Thus, the invention provides a method for discovering a disease biomarker comprising:
(i) providing a first cDNA sample from a subject with a disease and a second cDNA sample from a subject without the disease;
(ii) normalizing the first and the second cDNA samples;
(iii) sequencing the normalized first and second cDNA samples; and
(iv) comparing the sequencing output for the first and second cDNA samples to discover a disease biomarker;
   wherein the first and second cDNA samples comprise double stranded cDNA templates, each template having a known 5' pre-attached adapter and a known 3' pre-attached adapter;
   and wherein normalizing the first and second cDNA samples comprises:
      (i) denaturing the cDNA sample to produce single stranded cDNA templates;
      (ii) re-associating the cDNA sample to produce a mixture of post-association single stranded cDNA templates and post-association double stranded cDNA templates;
      (iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
      (iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
      (v) selectively amplifying the cDNA sample using primers specific to the ligated oligonucleotides.

Discovering a disease biomarker means identifying a novel biomarker (an indicator of a biological state) for a particular disease, for example uncovering a previously unknown biomarker for developing into a test for the disease. The disease biomarker may be suitable for use in diagnosing the disease, characterising the disease, predicting response to therapy, detecting minimal residual disease and/or prognosing the disease. By characterisation is meant classification and evaluation of the disease. Prognosis refers to predicting the likely outcome of the disease for the subject. In certain embodiments the characterisation of and/or prognosis for the disease comprises determining the grade and/or stage of the disease. In further embodiments the characterisation of the disease comprises determining the sub-type of the disease. The disease biomarker may be suitable for use in indicating the likelihood that a subject with a particular disease will benefit from a specific therapy.

In specific embodiments the disease is cancer. Thus, in certain embodiments the characterisation of and/or prognosis for the cancer comprises determining the presence or absence of metastases. Metastasis, or metastatic disease, is the spread of a cancer from one organ or part to another non-adjacent organ or part. The new occurrences of disease thus generated are referred to as metastases. Characterisation of and/or prognosis for the disease may also comprise predicting biochemical recurrence and/or determining whether the cancer is aggressive and/or determining whether the cancer has spread to the lymph nodes. Aggressive refers to a cancer that is fast growing, more likely to spread, more likely to recur and/or shows resistance to treatment.

Described herein is a method for monitoring a subject comprising:
(i) providing a first cDNA sample from the subject at a first time point and a second cDNA sample from the subject at a second time point;
(ii) normalizing the first and the second cDNA samples;
(iii) sequencing the normalized first and second cDNA samples; and
(iv) comparing the sequencing output for the first and second cDNA samples.

Monitoring a subject may comprise monitoring response to treatment for a disease. The first time point may be prior to starting treatment and the second time point may be during or after treatment. Comparing the sequencing output for the first and second cDNA samples may provide an indication as to whether treatment has been successful. For example, the presence or absence of a disease biomarker may indicate whether treatment has been successful. Comparing the sequencing output for the first and second cDNA samples may comprise comparing to each other and/or to the sequencing output from a reference sample.

According to all aspects of the invention the disease biomarker may be a cDNA sequence. The cDNA sequence will correspond to an RNA sequence. The cDNA/RNA sequence may correspond to a protein or peptide. In specific embodiments the method further comprises identifying an RNA, transcript model, gene, protein and/or peptide corresponding to a cDNA sequence. The disease biomarker may, therefore, be a cDNA molecule (of a specific sequence), DNA molecule (of a specific sequence), RNA molecule (of a specific sequence), transcript model, protein or peptide.

In specific embodiments the method comprises discovering more than one disease biomarker, optionally more than 10, 100, 1000, 10000, 100000, 1 million or 10 million disease biomarkers. In further embodiments the method comprises discovering between 1 and 10, 1 and 100, 1 and 1000, 1 and 10000, 1 and 100000, 1 and 1 million or 1 and 10 million disease biomarkers.

The disease biomarker may be a suitable target for a therapeutic agent, for example a vaccine, an RNA therapy and/or gene editing. The discovery of a disease biomarker specific to cancer cells can be an initial step in identifying a cancer specific antigen for a cancer vaccine to target. Thus, in specific embodiments, the method further comprises identifying a transcript or protein corresponding to the disease biomarker as a cancer vaccine target. The method may further comprise developing a cancer vaccine, optionally an RNA vaccine, directed to the target.

Described herein is a method for discovering a cancer vaccine target comprising:
(i) providing a first cDNA sample from a subject with a cancer and a second cDNA sample from a subject without the cancer;
(ii) normalizing the first and the second cDNA samples;
(iii) sequencing the normalized first and second cDNA samples; and
(iv) comparing the sequencing output for the first and second cDNA samples to discover a cancer vaccine target.

In specific embodiments the methods comprise providing 2, 3, 4, 5, 6, 7, 8, 9, 10, 20, 30, 40 or more cDNA samples from different subjects with the disease and/or providing 2, 3, 4, 5, 6, 7, 8, 9, 10, 20, 30, 40 or more cDNA samples from different subjects without the disease. Preferably, the methods comprise providing 30 or more cDNA samples from different subjects with the disease (e.g. with a cancer) and providing 30 or more cDNA samples from different subjects without the disease (e.g. without the cancer).

In specific embodiments the first cDNA sample and the second cDNA sample are obtained from biological fluid or a fluid or lysate generated from a biological material. The first cDNA sample and the second cDNA sample may be obtained from blood. The first cDNA sample may be obtained by extracting RNA from a biological sample (e.g. blood) obtained from the subject with the disease and the second cDNA sample may be obtained by extracting RNA from a biological sample (e.g. blood) obtained from the subject without the disease. cDNA is then synthesized using the RNA as a template (i.e. by reverse transcription). Thus, in specific embodiments the methods may further comprise, prior to step (i):
(a) extracting RNA from biological fluid or a fluid or lysate generated from a biological material from a subject with a disease and from a subject without the disease; and
(b) synthesizing cDNA using the RNA as a template (i.e. converting the RNA into cDNA). In this way the first cDNA sample and the second cDNA sample may be produced.

A subject with a disease means the subject has the disease at the time the biological sample (biological fluid or biological material) from which the cDNA sample is derived is taken from the subject. A subject without a disease means the subject does not have the disease at the time the biological sample (biological fluid or biological material) from which the cDNA sample is derived is taken from the subject.

The subject without the disease may be a healthy subject.

The disease biomarker may be a cDNA molecule (of a specific sequence), RNA molecule (of a specific sequence), protein or peptide that is detectable in a sample from a subject with a disease but not in a sample from a subject without the disease. Alternatively, the disease biomarker may be a cDNA molecule (of a specific sequence), RNA molecule (of a specific sequence), protein or peptide that is not detectable in a sample from a subject with a disease but is detectable in a sample from a subject without the disease.

The cancer vaccine target may be a cDNA molecule (of a specific sequence), RNA molecule (of a specific sequence), protein or peptide that is detectable in a sample from a subject with a cancer but not in a sample from a subject without the cancer.

In specific embodiments, the disease biomarker is a cDNA sequence that is present in the first cDNA sample but not in the second cDNA sample or is present in the second cDNA sample but not in the first cDNA sample. In specific embodiments, the disease biomarker is a transcript that is (uniquely) present in subjects with a particular disease. In further embodiments, the disease biomarker is a transcript that is (uniquely) absent in subjects with a particular disease.

In specific examples, the cancer vaccine target is a cDNA sequence that is present in the first cDNA sample but not in the second cDNA sample. In specific examples, the cancer vaccine target is a transcript that is uniquely present in subjects with a particular cancer. The transcript/cDNA sequence may correspond to a particular protein or peptide. At least a portion of the protein or peptide may form an antigen comprised in a cancer vaccine.

The present invention enables the identification of transcripts found only in subjects with a disease, optionally cancer. Such transcript models can be identified through comparison with subjects without the disease (e.g. benign patients) and, optionally, public transcriptome annotation databases. Sequencing output and/or resulting transcriptomic profile(s) from a subject with a particular disease (e.g. breast cancer) can be compared with sequencing output and/or resulting transcriptomic profile(s) from a subject with a different disease (for example, ovarian and/or colorectal cancer) to determine if the biomarker is unique to the particular disease (e.g. breast cancer).

In a further aspect the invention provides a method for diagnosing a disease in a subject comprising:
(i) providing a cDNA sample from the subject;
(ii) normalizing the cDNA sample; and
(iii) sequencing the normalized cDNA sample, wherein the sequencing output is used to identify whether the subject has the disease; wherein:
   the cDNA sample comprises double stranded cDNA templates, each template having a known 5' pre-attached adapter and a known 3' pre-attached adapter;
   and wherein normalizing the cDNA sample comprises:
      (i) denaturing the cDNA sample to produce single stranded cDNA templates;
      (ii) re-associating the cDNA sample to produce a mixture of post-association single stranded cDNA templates and post-association double stranded cDNA templates;
      (iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
      (iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
      (v) selectively amplifying the cDNA sample using primers specific to the ligated oligonucleotides;
   and wherein the method further comprises comparing the sequencing output for the normalized cDNA sample to one or more reference sequences or to the sequencing output of one or more control samples.

By diagnosing is meant determining that a subject has the disease at the time of testing.

In certain embodiments the disease is cancer and diagnosing the disease comprises detecting minimal residual disease (cancer cells that remain in the subject during or after treatment).

Described herein is a method for characterising and/or prognosing a disease in a subject comprising:
(i) providing a cDNA sample from the subject;
(ii) normalizing the cDNA sample; and
(iii) sequencing the normalized cDNA sample, wherein the sequencing output is used to provide a characterisation of and/or a prognosis for the disease.

Described herein is a method for selecting a treatment for a disease in a subject comprising:
(i) providing a cDNA sample from the subject;
(ii) normalizing the cDNA sample;
(iii) sequencing the normalized cDNA sample, wherein the sequencing output is used to provide a diagnosis, characterisation of and/or a prognosis for the disease; and
(iv) selecting a treatment appropriate to the diagnosis, characterisation of and/or prognosis for the disease.

Described herein is a method for predicting the responsiveness of a subject with a disease to a therapeutic agent comprising:
(i) providing a cDNA sample from the subject;
(ii) normalizing the cDNA sample; and
(iii) sequencing the normalized cDNA sample, wherein the sequencing output is used to predict the responsiveness of the subject to the therapeutic agent.

The method for diagnosing a disease in a subject comprises comparing the sequencing output for the normalized cDNA sample to one or more reference sequences or to the sequencing output of one or more control samples. The other methods herein may comprise comparing the sequencing output for the normalized cDNA sample to one or more reference sequences or to the sequencing output of one or more control samples. Optionally, the one or more control samples are from one or more subjects with and/or without the disease. Preferably, the methods comprise comparing the sequencing output for the normalized cDNA sample to the sequencing output of one or more control samples from one or more subjects with the disease.

By sequencing output is meant one or more sequences obtained from sequencing the (normalized) cDNA. The sequence(s) may be raw sequence(s) or may be further processed. For example, low quality reads may be filtered and/or adapter sequences may be filtered and removed. The (processed) sequence(s) may be mapped to the human reference genome (for example, using Minimap2) to prepare transcriptome profile(s). One or more transcript models may be identified in the transcriptome profile(s) (sequence(s) mapped to the genome). The transcript model represents a specific transcript i.e. a particular RNA isoform or splice variant produced from a gene. Thus, in specific embodiments, the sequencing output that is used in the methods defined herein (for example, that is compared to discover a disease biomarker or is used to identify whether the subject has a disease) may be transcriptome profile(s) and/or transcript model(s).

Using the sequencing output to identify whether the subject has the disease may comprise detecting a disease biomarker. In specific embodiments using the sequencing output to identify whether the subject has the disease comprises detecting more than one disease biomarker, optionally more than 10, 100, 1000, 10000, 100000, 1 million or 10 million disease biomarkers. In further embodiments using the sequencing output to identify whether the subject has the disease comprises detecting between 1 and 10, 1 and 100, 1 and 1000, 1 and 10000, 1 and 100000, 1 and 1 million or 1 and 10 million disease biomarkers. Detecting the disease biomarker may comprise determining the presence or absence of the disease biomarker. Thus, in specific embodiments, using the sequencing output to identify whether the subject has the disease comprises determining the presence or absence of more than one disease biomarker, optionally more than 10, 100, 1000, 10000, 100000, 1 million or 10 million disease biomarkers. In further embodiments using the sequencing output to identify whether the subject has the disease comprises determining the presence or absence of between 1 and 10, 1 and 100, 1 and 1000, 1 and 10000, 1 and 100000, 1 and 1 million or 1 and 10 million disease biomarkers.

The presence of a particular cDNA sequence in the sequencing output may indicate that the subject has the disease, for example where a particular transcript (corresponding to the cDNA molecule) is uniquely present in subjects with a particular disease. Likewise, the presence of a particular cDNA sequence in the sequencing output may indicate a characterisation of and/or a prognosis for the disease. The presence of a particular cDNA sequence in the sequencing output may allow prediction of the responsiveness of a subject with a disease to a therapeutic agent, for example where a particular transcript has been found to correlate with responsiveness of a subject with a disease to a particular therapeutic agent.

The absence of a particular cDNA sequence in the sequencing output may indicate that the subject has the disease, for example where a particular transcript (corresponding to the cDNA molecule) is absent in subjects with a particular disease. Likewise, the absence of a particular cDNA sequence in the sequencing output may indicate a characterisation of and/or a prognosis for the disease. The absence of a particular cDNA sequence in the sequencing output may allow prediction of the responsiveness of a subject with a disease to a therapeutic agent, for example where a particular transcript has been found to correlate with responsiveness of a subject with a disease to a particular therapeutic agent.

According to all aspects of the invention, in specific embodiments the cDNA sample is obtained from a biological fluid or a fluid or lysate generated from a biological material. The cDNA sample may be obtained from blood. The cDNA sample may be obtained by extracting RNA from a biological sample (e.g. blood) obtained from the subject. cDNA is then synthesized using the RNA as a template (i.e. by reverse transcription). Thus, in certain embodiments the methods further comprise, prior to step (i):
(a) extracting RNA from biological fluid or a fluid or lysate generated from a biological material from the subject; and
(b) synthesizing cDNA using the RNA as a template (i.e. converting the RNA into cDNA).

In this way the cDNA sample from the subject may be produced.

The methods may further comprise reporting to the subject the outcome of the method. The result may be a diagnosis or prognosis for the disease. In specific embodiments the result is a specific grade or stage of a disease, such as a cancer.

Complementary DNA (cDNA) normalization (Alex S. Shcheglov, Pavel A. Zhulidov, Ekaterina A. Bogdanova, D. A. S. Normalization of cDNA Libraries, Nucleic Acids Hybrid. CHAPTER 5, (2014)) addresses issues with high abundance house-keeping genes reducing sampling efficiency for genes of interest. Since RNA sequencing typically relies on the conversion of RNA to double stranded cDNA, cDNA normalization takes advantage of the biochemical properties of cDNA to generate a uniform distribution of unique genes and isoforms within a cDNA library. In theory, the maximum non-targeted sampling efficiency is produced if all unique RNA sequences are represented at the same relative abundance. Thus, the objective of normalization is to re-distribute a cDNA library (sample) to meet this criterion as closely as possible.

Normalizing a cDNA sample results in production of a normalized cDNA sample. In certain embodiments, normalizing comprises (selectively) increasing the relative abundance of less abundant sequences without targeting specific sequences based on their nucleotide sequence (i.e. identity or homology to a known sequence).

By "normalized" is meant that the levels of RNA or cDNA sequences in the sample are more equal. Thus, a normalized cDNA sample may be one in which the amount of each unique cDNA sequence is more uniform than in the same sample prior to normalization i.e. a normalized cDNA sample is closer to achieving each unique cDNA sequence having the same abundance (relative to other unique cDNA sequences within the normalized cDNA sample) than the same sample prior to normalization. To achieve this the relative representation or levels of less abundant sequences may be increased and/or the relative representation or levels of more abundant sequences may be decreased. The increase in less abundant sequences/decrease in more abundant sequences is selective in the sense that if all sequences were increased/decreased to the same degree the relative abundance would stay the same. However, the relative representation or levels of less abundant sequences may be increased and/or the relative representation or levels of more abundant sequences may be decreased without targeting (for example, using pre-defined probes) specific sequences based on their nucleotide composition (i.e. based on their identity or homology to a known sequence). According to all embodiments, the less abundant sequences may be the unique sequences with an amount that is below a threshold, for example they are present in the cDNA sample prior to normalization in an amount that is below the mean amount for a unique sequence in the sample. The less abundant sequences may be present in the cDNA sample prior to normalization at an amount that is 0.1%, 1%, 10%, 20%, 30%, 40%, 50%, 60%, 70, 80% or 90% below the mean amount for a unique sequence in the sample. The more abundant sequences may be the unique sequences with an amount that is above a threshold, for example they are present in the cDNA sample prior to normalization in an amount that is above the mean amount for a unique sequence in the sample. The more abundant sequences may be present in the cDNA sample prior to normalization at an amount that is 0.1%, 1%, 10%, 20%, 30%, 40%, 50%, 60%, 70, 80% or 90% above the mean amount for a unique sequence in the sample. By relative abundance is meant abundance relative to other unique sequences in the sample.

In specific embodiments a normalized cDNA sample comprises cDNA sequences having substantially the same levels. For example, wherein the levels of the sequences of the normalized cDNA vary by less than 50%, less than 40%, less than 30%, less than 20%, or less than 10%. The normalized cDNA may be a normalized cDNA sample in which at least a portion of the 10, 100, 1000, or 10000 most abundant (unique) sequences in the cDNA sample have been removed or reduced (by at least 1%, 10%, 20%, 30%, 40%, 50%, 60%, 70%, 80% or 90%) in copy number. The normalized cDNA may be a normalized cDNA sample in which levels of at least a portion of the 10, 100, 1000, or 10000 least abundant (unique) sequences in the cDNA sample have been increased e.g. by at least 1%, 10%, 20%, 30%, 40%, 50%, 60%, 70%, 80% or 90% in copy number. The methods for normalizing cDNA sample(s) described herein may be methods for equalizing cDNA sample(s) i.e. equalizing the relative abundances of each unique sequence.

According to all aspects of the invention, in specific embodiments normalizing a cDNA sample reduces the variability in the levels of the cDNA (e.g. by at least 10%, at least 20%, at least 30%, at least 40%, at least 50%, at least 60%, at least 70%, at least 80%, or at least 90%). Normalizing cDNA may achieve a more uniform distribution of cDNA sequences. The difference in abundance between the most abundant cDNA and the least abundant cDNA in the sample may be reduced (e.g. by at least 10%, at least 20%, at least 30%, at least 40%, at least 50%, at least 60%, at least 70%, at least 80%, or at least 90%). In certain embodiments normalizing the cDNA sample reduces the number of molecules (copy number) of the (1, 10, 100, 1000, or 10000) most abundant cDNA molecule(s) by at least 10%, at least 20%, at least 30%, at least 40%, at least 50%, at least 60%, at least 70%, at least 80%, or at least 90%. In specific embodiments the number of molecules (copy number) of the most abundant cDNA molecule in the (first and/or second) cDNA sample is reduced by at least 50% in the normalized cDNA. In further embodiments the relative abundance of the (1, 10, 100, 1000, or 10000) least abundant cDNA molecule(s) is increased by at least 10%, at least 20%, at least 30%, at least 40%, at least 50%, at least 60%, at least 70%, at least 80%, or at least 90%. In specific embodiments the number of molecules (copy number) of the least abundant cDNA molecule in the (first and/or second) cDNA sample is increased by at least 50% in the normalized cDNA.

According to all aspects of the invention, in certain embodiments normalizing the (first and/or the second) cDNA sample(s) increases the amount (copy number) of at least a portion of the low abundance cDNA sequences within the (first and/or the second) cDNA sample(s). The low abundance cDNA sequences may be the 50%, 40%, 30%, 20%, 10% or 1% of (unique) sequences with the lowest copy number. Thus, normalizing may comprise selectively increasing the amount of low abundance cDNA within each cDNA sample.

In certain embodiments normalized cDNA is cDNA that is more readily analysable. It may be more efficiently sequenced because the relative representation of less abundant sequences is increased. In specific embodiments normalizing the (first and/or the second) cDNA sample(s) does not comprise removing abundant (more abundant) cDNA molecules/sequences (such as those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin) from the sample(s) (for example, using duplex-specific nuclease or sequence targeted methods). In further embodiments normalizing does not comprise targeting specific (unique) sequences (such as those corresponding to Albumin, IgG, Apolipoprotein A-I, Transferrin, Apolipoprotein A-II, α₁-Proteinase inhibitor, α₁-Acid glycoprotein, Transthyretin, Hepatoglobin and/or Hemopexin). Thus, in specific embodiments normalizing a cDNA sample is non-targeted i.e. it does not involve targeting specific sequences based on their nucleotide sequence (for example, it does not involve targeting a particular sequence based on its identity or homology to a known sequence).

According to all aspects of the invention, the term "sequence" may refer to all of the individual nucleic acid (e.g. cDNA or RNA) molecules having a 100% identical nucleotide sequence. Alternatively, the term "sequence" may refer to all of the individual nucleic acid (e.g. cDNA or RNA) molecules having more than 99%, 98%, 97%, 96%, 95%, 90%, 85%, 80%, 75%, 70%, 65%, 60%, 55% or 50% identity to one another. "% identity" between a query nucleic acid sequence and a subject nucleic acid sequence may be calculated using a suitable algorithm (e.g. BLASTN, FASTA, Needleman-Wunsch, Smith-Waterman, LALIGN, or GenePAST/KERR) or software (e.g. DNASTAR Lasergene, GenomeQuest, EMBOSS needle or EMBOSS infoalign), over the entire length of the query sequence after a pair-wise global sequence alignment has been performed using a suitable algorithm (e.g. Needleman-Wunsch or GenePAST/KERR) or software (e.g. DNASTAR Lasergene or GenePAST/KERR). The term "unique sequence" or "unique cDNA sequence" may refer to all of the individual nucleic acid (e.g. cDNA or RNA as appropriate) molecules which meet or exceed a threshold % identity (e.g. 100%, 99%, 98%, 97%, 96%, 95%, 90%, 85%, 80%, 75%, 70%, 65%, 60%, 55% or 50% identity to one another). The "unique sequence" or "unique cDNA sequence" may differ from the other sequences present in the sample (for example, by at least 1, 2, 3, 4, 5, 6, 7, 8, 9, 10, 20, 50 or 100 nucleotides or by at least 1%, 2%, 3%, 4%, 5%, 10%, 15%, 20%, 25%, 30%, 35%, 40%, 45% or 50% of their sequence).

According to all aspects of the invention, in specific embodiments the disease is not an infectious disease. In certain embodiments the disease is cancer. The cancer may be an epithelial cancer. In specific embodiments, the cancer is breast, ovarian and/or colorectal cancer. Preferably, the cancer is breast cancer. In further embodiments the first cDNA sample is from a subject with breast cancer and the second cDNA sample is from a subject with a benign breast condition.

In particular embodiments the method can be used to diagnose more than one disease in a single process, for example through detection of multiple cDNA molecules (derived from transcripts) that are each uniquely present in subjects with a particular disease.

In specific embodiments the method can be used to diagnose more than one cancer type. In certain embodiments the method can be used to distinguish between breast cancer and a benign breast condition.

The present inventors have developed a selective amplification method (termed "Level-Up") that can be used for cDNA normalization. Level-Up normalization makes it possible to take a cDNA library and equalize the relative abundances of each unique transcript sequence. This is done without depletion and without targeting. In essence this allows creation of an optimal cDNA library for detecting all RNA that are present in the sample.

Thus, normalizing a cDNA sample may be achieved by using a method of selective amplification of single stranded cDNA, the method comprising:
(i) providing a cDNA sample comprising double stranded cDNA templates, each template having a known 5' pre-attached adapter and a known 3' pre-attached adapter;
(ii) denaturing the cDNA sample to produce single stranded cDNA templates;
(iii) re-associating the cDNA sample to produce a mixture of post-association single stranded cDNA templates and post-association double stranded cDNA templates;
(iv) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(v) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(vi) selectively amplifying the cDNA sample using primers specific to the ligated oligonucleotides.

Thus, in some aspects the first and second cDNA samples comprise double stranded cDNA templates, each template having a known 5' pre-attached adapter and a known 3' pre-attached adapter;
and normalizing the first and second cDNA samples comprises:
(i) denaturing the cDNA sample to produce single stranded cDNA templates;
(ii) re-associating the cDNA sample to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA sample using primers specific to the ligated oligonucleotides.

In further aspects the cDNA sample comprises double stranded cDNA templates, each template having a known 5' pre-attached adapter and a known 3' pre-attached adapter;
and normalizing the cDNA sample comprises:
(i) denaturing the cDNA sample to produce single stranded cDNA templates;
(ii) re-associating the cDNA sample to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA sample using primers specific to the ligated oligonucleotides.

The invention also provides a method for discovering a disease biomarker comprising:
(i) providing a first cDNA sample from a subject with a disease and a second cDNA sample from a subject without the disease, wherein the first and second cDNA samples comprise double stranded cDNA templates, each template having a known 5' pre-attached adapter and a known 3' pre-attached adapter;
(ii) denaturing the first and second cDNA samples to produce single stranded cDNA templates;
(iii) re-associating the first and second cDNA samples to produce a mixture of post-association single stranded cDNA templates and post-association double stranded cDNA templates;
(iv) within each of the first and second cDNA samples annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(v) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template;
(vi) selectively amplifying the first and second cDNA samples using primers specific to the ligated oligonucleotides
(vii) sequencing the selectively amplified first and second cDNA samples; and
(viii) comparing the sequencing output for the first and second cDNA samples to discover a disease biomarker.

The first and second cDNA samples are kept separate and/or are separately identifiable.

The invention further provides a method for diagnosing a disease in a subject, the method comprising:
(i) providing a cDNA sample from a subject comprising double stranded cDNA templates, each template having a known 5' pre-attached adapter and a known 3' pre-attached adapter;
(ii) denaturing the cDNA sample to produce single stranded cDNA templates;
(iii) re-associating the cDNA sample to produce a mixture of post-association single stranded cDNA templates and post-association double stranded cDNA templates;
(iv) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(v) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(vi) selectively amplifying the cDNA sample using primers specific to the ligated oligonucleotides; and
(vii) sequencing the selectively amplified cDNA sample, wherein the sequencing output is used to identify whether the subject has the disease.

In certain embodiments:
(A) the 5' adapter complex is a front oligonucleotide dimer comprising:
   (i) a front lig-oligonucleotide for ligating to the 5' pre-attached adapter of the (post-association) single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) the 3' adapter complex is a back oligonucleotide dimer comprising:
   (i) a back lig-oligonucleotide for ligating to the 3' pre-attached adapter of the (post-association) single stranded cDNA template; and
   (ii) a back link-oligonucleotide for annealing to the 3' pre-attached adapter and the back lig-oligonucleotide, the back link-oligonucleotide comprising a region complementary to the 3' pre-attached adapter and a region complementary to the back lig-oligonucleotide,
such that, on annealing, an end of the back lig-oligonucleotide is adjacent an end of the 3' pre-attached adapter to enable ligation of the back lig-oligonucleotide to the 3' pre-attached adapter at a ligation site.

Suitably:
(A) the front link-oligonucleotide comprises:
   (i) a template overhang region at an end of the front link-oligonucleotide proximal the region complementary to the 5' pre-attached adapter, the template overhang region being non-complementary to a corresponding region of the (post-association) single stranded cDNA template; and/or
   (ii) a lig-oligonucleotide overhang region at an end of the front link-oligonucleotide proximal the region complementary to the front lig-oligonucleotide, the lig-oligonucleotide overhang region being non-complementary to a corresponding region of the front lig-oligonucleotide; and/or
(B) the back link-oligonucleotide comprises:
   (i) a template overhang region at an end of the back link-oligonucleotide proximal the region complementary to the 3' pre-attached adapter, the template overhang region being non-complementary to a corresponding region of the (post-association) single stranded cDNA template; and/or
   (ii) a lig-oligonucleotide overhang region at an end of the back link-oligonucleotide proximal the region complementary to the back lig-oligonucleotide, the lig-oligonucleotide overhang region being non-complementary to a corresponding region of the back lig-oligonucleotide.

Suitably, the template overhang and/or lig-oligonucleotide overhang is between about 1 bp and about 20 bp in length. The template overhang and/or lig-oligonucleotide overhang may be between 2 bp and 19 bp, between 3 bp and 18 bp, between 2 bp and 17 bp, between 3 bp and 16 bp, between 2 bp and 15 bp, between 3 bp and 14 bp, between, 2 bp and 13 bp, between 3 bp and 12 bp, between 2 bp and 11 bp, between 3 bp and 10 bp, between 2 bp and 9 bp, between 3 bp and 8 bp, between 2 bp and 7 bp, between 3 bp and 6 bp, between 2 bp and 5 bp, between 3 bp and 5 bp or between 2 bp and 4 bp. Preferably, the template overhang and/or lig-oligonucleotide overhang is 3 bp.

**The** template overhang and/or lig-oligonucleotide overhang may be at least 2 bp, or at least 3 bp. Preferably, the template overhang and/or lig-oligonucleotide overhang is at least 3 bp.

Suitably, a combined length of the front link-oligonucleotide and the front lig-oligonucleotide is less than about 300 bp and/or a combined length of the back link-oligonucleotide and the back lig-oligonucleotide is less than about 300 bp. A combined length of the front link-oligonucleotide and the front lig-oligonucleotide may be at least about 200 bp and/or a combined length of the back link-oligonucleotide and the back lig-oligonucleotide may be at least about 100 bp.

Suitably, the front and/or back link-oligonucleotide has a length of less than 200 bp. The front and/or back link-oligonucleotide may have a length of at least 50 bp.

Suitably, the front oligonucleotide dimer and/or the back oligonucleotide dimer has at least one non-blunt end.

Suitably, the front link-oligonucleotide and/or the back link-oligonucleotide provides at least 5 bp of complementary binding either side of the ligation site.

Suitably, a nucleotide sequence of the front oligonucleotide dimer is different and non-complementary to a nucleotide sequence of the back oligonucleotide dimer.

Suitably, at least one of the front oligonucleotide dimer and the back oligonucleotide dimer is annealable to the (post-association) single stranded cDNA template at a temperature of over 30°C.

Suitably, a concentration of the front oligonucleotide dimer and/or a concentration of the back oligonucleotide dimer exceeds a concentration of a predicted total single stranded cDNA concentration or total cDNA in the cDNA sample.

Suitably, the step of re-associating the cDNA sample has a duration of 0-24 hours, optionally 0-8 hours, 1-7 hours, 1-24 hours or 7-24 hours.

By using long read sequencing, it is possible to detect full length RNA/cDNA which will provide better information for identifying the tissue source of each RNA and the specific function. While long read RNA sequencing is expensive compared to other assays, Level-Up technology makes it possible to reduce the amount of sequencing required thus lowering the overall cost.

Thus, according to all aspects of the invention, in specific embodiments sequencing comprises the use of long read sequencing. Thus, sequencing may be by long-read sequencing, for example long read sequencing allowing for sequencing reads of more than 1000, 5000 or 10000bp.

Level-Up makes it possible to analyze samples with RNA degradation by increasing the presence of low abundance transcripts. However, it is preferable if RNA degradation is minimized during the processing of biological samples to produce cDNA samples. The present inventors have developed a method for processing a blood sample when RNA extraction is not carried out on the day of blood collection. Specific steps for blood sample freezing, storage and thawing improve the condition of samples, particularly if they are to be subjected to long read sequencing.

Accordingly, disclosed herein is a method for processing a blood sample comprising:
(i) storing the blood sample at -15°C or below;
(ii) thawing the blood sample at 5 to 30°C for at least 1 hour; and
(iii) extracting RNA from the thawed blood sample.

The blood sample may be a liquid (i.e. non-dried) blood sample. The blood sample may be whole blood. In certain embodiments the blood sample is in a sample tube. In specific embodiments the blood sample is not absorbed into a material such as a sponge.

In certain embodiments the blood sample is stored at between -15°C and -80°C, -15°C and -70°C, -15°C and -60°C, -15°C and -50°C, -15°C and -40°C, -15°C and -30°C or -15°C and -20°C.

The blood sample may be stored at -15°C or below within 12 hours, 8 hours, 5 hours, 2 hours, 1 hour, 30 minutes, 15 minutes, 5 minutes or 1 minute of collection. Preferably, the blood sample is stored at -15°C or below within 12 hours of collection (i.e. taking the blood sample from the subject).

In certain embodiments the blood sample is stored at -20°C or below. The blood sample may be stored at -20°C or below within 12 hours, 8 hours, 5 hours, 2 hours, 1 hour, 30 minutes, 15 minutes, 5 minutes or 1 minute of collection. Preferably, the blood sample is stored at -20°C or below within 12 hours of collection (i.e. taking the blood sample from the subject).

In certain embodiments the blood sample is stored at -15°C or below (optionally -20°C or below) for at least 24 hours (and optionally for no more than 72 hours, 1 week, 2 weeks, 4 weeks, 1 month or 2 months) before storing at -70°C or below (optionally -80°C or below) for no more than 4, 5, 6, 7, 8, 9, 10, 11, or 12 months or 2, 3, 4 or 5 years. Preferably, storage at -70°C or below (optionally -80°C or below) is for no more than 5 years.

Preferably, thawing of the blood sample takes place on the same day RNA is to be extracted. In specific embodiments the blood sample is thawed at 16 to 29°C, 17 to 28°C, 18 to 27°C, 18 to 26°C or 18 to 25°C. Preferably, the blood sample is thawed at 18 to 25°C. The duration of the thawing step may be 1 to 5 hours, 2 to 5 hours, 1 to 4 hours, 2 to 4 hours, 1 to 3 hours, 2 to 3 hours or 1 to 2 hours. In a preferred embodiment the blood sample is thawed at 18 to 25°C for 1 to 3 hours. More preferably, the blood sample is thawed at 18 to 25°C for 3 hours.

In specific embodiments once the blood sample is fully thawed the sample tube is inverted at least 5, 6, 7, 8, 9 or 10 times. Preferably, the sample tube is inverted 10 times. The blood sample may then be incubated at 18 to 25°C for around 2 hours prior to RNA extraction.

RNA may be extracted from the (thawed) blood sample using the Qiagen Paxgene Blood RNA Kit.

In specific embodiments, prior to step (i), the blood is received in a container (optionally a Paxgene Blood RNA Tube) at room temperature (5 to 30°C, preferably 18 to 25°C). The container may be inverted at least 5, 6, 7, 8, 9 or 10 times immediately after blood collection. Preferably, the container is inverted 10 times immediately after blood collection. The blood sample may be stored at-15°C or below (or -20°C or below) immediately after being inverted.

Thus, disclosed herein is a method for processing a blood sample comprising:
(i) receiving the blood sample in a container at 18 to 25°C;
(ii) storing the blood sample at -20°C or below within 12 hours of collection;
(iii) thawing the blood sample at 18 to 25°C for 1 to 3 hours; and
(iv) extracting RNA from the thawed blood sample.

According to all aspects of the invention, in particular embodiments, the blood sample is no more than 5 ml, 3 ml, 2.5 ml or 2 ml. Preferably, the blood sample is no more than 2.5 ml. The blood sample may be between 0.1 ml and 5 ml, 0.5 ml and 5 ml, 1 ml and 4 ml, or 2 ml and 3 ml.

The methods for processing a blood sample may be combined with the methods outlined above that employ a cDNA sample. Thus, in specific embodiments, the cDNA sample is obtained from blood by following the steps of the methods for processing a blood sample described herein. In addition, the method may comprise a step of synthesizing cDNA using the extracted RNA as a template (i.e. converting the extracted RNA into cDNA, reverse transcription). In further embodiments the first cDNA sample and the second cDNA sample are obtained from blood by following the steps of the methods for processing a blood sample described herein. In addition, the method may comprise a step of synthesizing cDNA using the extracted RNA as a template (i.e. converting the extracted RNA into cDNA, reverse transcription).

In some embodiments, the cDNA sample is obtained from blood by a method for processing a blood sample comprising:
(i) storing the blood sample at -15°C or below;
(ii) thawing the blood sample at 5 to 30°C for at least 1 hour;
(iii) extracting RNA from the thawed blood sample; and
(iv) synthesizing cDNA using the extracted RNA as a template.

In some embodiments, the first cDNA sample and the second cDNA sample are obtained from blood by a method for processing a blood sample comprising:
(i) storing the blood sample at -15°C or below;
(ii) thawing the blood sample at 5 to 30°C for at least 1 hour;
(iii) extracting RNA from the thawed blood sample; and
(iv) synthesizing cDNA using the extracted RNA as a template.

In some embodiments, the blood sample is stored at -15°C or below or -20°C or below within 12 hours of collection
Described herein is a method for discovering a disease biomarker comprising:
(i) receiving a first blood sample from a subject with a disease in a first container and a second blood sample from a subject without the disease in a second container at 18 to 25°C;
(ii) storing the first and second blood samples at -20°C or below within 12 hours of collection from the subjects;
(iii) thawing the first and second blood samples at 18 to 25°C for 1 to 3 hours;
(iv) extracting RNA from the thawed first and second blood samples;
(v) synthesizing cDNA using the extracted RNA as a template to form a first cDNA sample from the subject with the disease and a second cDNA sample from the subject without the disease;
(vi) normalizing the first and the second cDNA samples;
(vii) sequencing the normalized first and second cDNA samples; and
(viii) comparing the sequencing output for the first and second cDNA samples to discover a disease biomarker.

Described herein is a method for diagnosing a disease in a subject comprising:
(i) receiving a blood sample from the subject in a container at 18 to 25°C;
(ii) storing the blood sample at -20°C or below within 12 hours of collection from the subject;
(iii) thawing the blood sample at 18 to 25°C for 1 to 3 hours;
(iv) extracting RNA from the thawed blood sample;
(v) synthesizing cDNA using the extracted RNA as a template to form a cDNA sample from the subject;
(vi) normalizing the cDNA sample; and
(vii) sequencing the normalized cDNA sample, wherein the sequencing output is used to identify whether the subject has the disease.

A further aspect of the present invention provides use in a method for discovering a cancer biomarker of an oligonucleotide dimer composition for selective amplification of single stranded cDNA by ligation of an oligonucleotide to a 5' and a 3' end of a post-association single stranded cDNA template having known 5' and 3' pre-attached adapters, wherein the composition comprises:
(A) a front oligonucleotide dimer comprising:
   (i) a front lig-oligonucleotide for ligating to the 5' pre-attached adapter of the post-association single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
   (i) a back lig-oligonucleotide for ligating to the 3' pre-attached adapter of the post-association single stranded cDNA template; and
   (ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
   such that, on annealing, an end of the back lig-oligonucleotide is adjacent an end of the 3' pre-attached adapter to enable ligation of the back lig-oligonucleotide to the 3' pre-attached adapter at a ligation site.

Described herein is use in a method for discovering a cancer vaccine target of an oligonucleotide dimer composition for selective amplification of single stranded cDNA by ligation of an oligonucleotide to a 5' and a 3' end of a post-association single stranded cDNA template having known 5' and 3' pre-attached adapters, wherein the composition comprises:
(A) a front oligonucleotide dimer comprising:
   (i) a front lig-oligonucleotide for ligating to the 5' pre-attached adapter of the post-association single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
   (i) a back lig-oligonucleotide for ligating to the 3' pre-attached adapter of the post-association single stranded cDNA template; and
   (ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
   such that, on annealing, an end of the back lig-oligonucleotide is adjacent an end of the 3' pre-attached adapter to enable ligation of the back lig-oligonucleotide to the 3' pre-attached adapter at a ligation site.

Described herein is use in a method for diagnosing and/or prognosing a cancer in a subject of an oligonucleotide dimer composition for selective amplification of single stranded cDNA by ligation of an oligonucleotide to a 5' and a 3' end of a post-association single stranded cDNA template having known 5' and 3' pre-attached adapters, wherein the composition comprises:
(A) a front oligonucleotide dimer comprising:
   (i) a front lig-oligonucleotide for ligating to the 5' pre-attached adapter of the post-association single stranded cDNA template; and
   (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
   such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
   (i) a back lig-oligonucleotide for ligating to the 3' pre-attached adapter of the post-association single stranded cDNA template; and
   (ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
   such that, on annealing, an end of the back lig-oligonucleotide is adjacent an end of the 3' pre-attached adapter to enable ligation of the back lig-oligonucleotide to the 3' pre-attached adapter at a ligation site.

The oligonucleotide dimer composition as defined herein may also be used in a method for characterising a disease in a subject, a method for selecting a treatment for a disease in a subject and/or a method for predicting the responsiveness of a subject with a disease to a therapeutic agent.

In specific embodiments:
(A) the front link-oligonucleotide comprises:
   (i) a template overhang region at an end of the front link-oligonucleotide proximal the region complementary to the 5' pre-attached adapter, the template overhang region being non-complementary to a corresponding region of the post-association single stranded cDNA template; and/or
   (ii) a lig-oligonucleotide overhang region at an end of the front link-oligonucleotide proximal the region complementary to the front lig-oligonucleotide, the lig-oligonucleotide overhang region being non-complementary to a corresponding region of the front lig-oligonucleotide; and/or
(B) the back link-oligonucleotide comprises:
   (i) a template overhang region at an end of the back link-oligonucleotide proximal the region complementary to the 3' pre-attached adapter, the template overhang region being non-complementary to a corresponding region of the post-association single stranded cDNA template; and/or
   (ii) a lig-oligonucleotide overhang region at an end of the back link-oligonucleotide proximal the region complementary to the back lig-oligonucleotide, the lig-oligonucleotide overhang region being non-complementary to a corresponding region of the back lig-oligonucleotide.

Suitably, the template overhang and/or lig-oligonucleotide overhang is between about 1 bp and about 20 bp in length. The template overhang and/or lig-oligonucleotide overhang may be between 2 bp and 19 bp, between 3 bp and 18 bp, between 2 bp and 17 bp, between 3 bp and 16 bp, between 2 bp and 15 bp, between 3 bp and 14 bp, between, 2 bp and 13 bp, between 3 bp and 12 bp, between 2 bp and 11 bp, between 3 bp and 10 bp, between 2 bp and 9 bp, between 3 bp and 8 bp, between 2 bp and 7 bp, between 3 bp and 6 bp, between 2 bp and 5 bp, between 3 bp and 5 bp or between 2 bp and 4 bp. Preferably, the template overhang and/or lig-oligonucleotide overhang is 3 bp.

The template overhang and/or lig-oligonucleotide overhang may be at least 2 bp, or at least 3 bp. Preferably, the template overhang and/or lig-oligonucleotide overhang is at least 3 bp.

Suitably, a combined length of the front link-oligonucleotide and the front lig-oligonucleotide is less than about 300 bp and/or a combined length of the back link-oligonucleotide and the back lig-oligonucleotide is less than about 300 bp.

Suitably, the front and/or back link-oligonucleotide has a length of less than 200 bp.

Suitably, the front oligonucleotide dimer and/or the back oligonucleotide dimer has at least one non-blunt end.

Suitably, in use of the composition, the front link-oligonucleotide and/or the back link-oligonucleotide provides at least 5 bp of complementary binding either side of the ligation site.

Suitably, a nucleotide sequence of the front oligonucleotide dimer is different and non-complementary to a nucleotide sequence of the back oligonucleotide dimer.

Suitably, the front oligonucleotide dimer and/or the back oligonucleotide dimer is annealable to the post-association single stranded cDNA template at a temperature of over 30°C.

A selective amplification kit is described herein for selectively amplifying low abundance cDNA from a cDNA sample and/or for selective amplification of cDNA comprising known adapter sequences, the cDNA sample comprising cDNA templates having known 5' and 3' pre-attached adapters, the kit comprising means for preparing an oligonucleotide dimer composition as described above and means for implementing the method of selective amplification as described above.

Described herein is use of kit as described herein in a method for diagnosing and/or prognosing a disease in a subject. Also described herein is use of kit as described herein in a method for characterising a disease in a subject, a method for selecting a treatment for a disease in a subject and/or a method for predicting the responsiveness of a subject with a disease to a therapeutic agent.

A selective amplification kit is also described herein for selectively amplifying low abundance cDNA from a first cDNA sample from a subject with a disease and a second cDNA sample from a subject without the disease and/or for selective amplification of cDNA comprising known adapter sequences, the first and second cDNA samples comprising cDNA templates having known 5' and 3' pre-attached adapters, the kit comprising means for preparing an oligonucleotide dimer composition as described above and means for implementing the method of selective amplification as described above.

Described herein is use of kit as described herein in a method for discovering a disease biomarker and/or a cancer vaccine target.

The means for preparing an oligonucleotide dimer composition may comprise a front lig-oligonucleotide, a front link-oligonucleotide, a back lig-oligonucleotide and/or a back link-oligonucleotide as described herein. The means for preparing an oligonucleotide dimer composition may comprise a front oligonucleotide dimer and/or a back oligonucleotide dimer as described herein.

The means for implementing the method of selective amplification may comprise primers specific to the front and/or back lig-oligonucleotides.

The kit may further comprise a hybridization buffer. The hybridization buffer may comprise HEPES 1M (pH = 7.5), NaCl 5M and H₂0. The kit may also comprise ligase and/or ligase buffer. Any suitable ligase may be used. The ligase may be a nick repair ligase or a blunt end ligase. Optionally, the ligase may be Taq DNA ligase. Suitable ligase buffers are also well known and commercially available.

The kit may further comprise primers for adding phosphate groups to cDNA prior to its use as a cDNA sample. These primers are based on the known 5' pre-attached adapter and known 3' pre-attached adapter sequences.

The kit may further comprise suitable reagents for PCR including one or more, up to all, of a polymerase, dinucleotide triphosphates (dNTPs), MgCl₂ and buffer. Any suitable polymerase may be utilised. Generally, DNA polymerases are used to amplify nucleic acid targets according to the invention. Examples include thermostable polymerases such as Taq or Pfu polymerase and the various derivatives of those enzymes. Suitable buffers are also well known and commercially available and may be included in a PCR mastermix that includes the majority of the components required for PCR amplification.

The kit may further comprise suitable reagents for reverse transcription of RNA to cDNA including a reverse transcriptase enzyme. Any suitable reverse transcriptase may be utilised. Suitable buffers are also well known and commercially available and may be included in a reverse transcription mastermix that includes the majority of the components required for reverse transcription.

The kit may further comprise suitable reagents for processing a blood sample, for example a container (optionally a PAXgene Blood RNA tube), RNA stabilizing reagent and/or blood cell lysis buffer. The reagents may be RNase-free. RNA stabilizing reagents are commercially available and include RNAlater^{®} (Sigma-Aldrich) and RNAprotect (Qiagen). A suitable RNA stabilizing reagent may comprise EDTA, sodium citrate and/or ammonium sulfate, for example 70% (w/v) Ammonium Sulfate, 25 mM Sodium Citrate and/or 10 mM EDTA. The pH may be adjusted to 5.2 with sulfuric acid.

Described herein is use of a set of reagents in a method as described herein, the set of reagents comprising:
a front lig-oligonucleotide, a front link-oligonucleotide, a back lig-oligonucleotide and/or a back link-oligonucleotide; and
primers specific to the front and/or back lig-oligonucleotides.

The front lig-oligonucleotide, front link-oligonucleotide, back lig-oligonucleotide and/or back link-oligonucleotide may be provided as an oligonucleotide dimer composition as described herein.

The set of reagents may further comprise one or more up to all of the following: a hybridization buffer (optionally comprising HEPES 1M (pH = 7.5), NaCl 5M and H₂0), a ligase, a ligase buffer, a primer pair for adding phosphate groups to cDNA, a DNA polymerase and/or dNTPs.

In further examples of the disclosure, the set of reagents further comprises suitable reagents for processing a blood sample, for example a container (optionally a PAXgene Blood RNA tube), RNA stabilizing reagent and/or blood cell lysis buffer. The reagents may be RNase-free. RNA stabilizing reagents are commercially available and include RNAlater^{®} (Sigma-Aldrich) and RNAprotect (Qiagen). A suitable RNA stabilizing reagent may comprise EDTA, sodium citrate and/or ammonium sulfate, for example 70% (w/v) Ammonium Sulfate, 25 mM Sodium Citrate and/or 10 mM EDTA. The pH may be adjusted to 5.2 with sulfuric acid. Incubation for cell lysis can be, for example, 1 minute to 3 hours.

In an example of the disclosure, the set of reagents comprises:
a RNA stabilizing reagent;
a (blood) cell lysis buffer;
a front lig-oligonucleotide, a front link-oligonucleotide, a back lig-oligonucleotide and/or a back link-oligonucleotide;
primers specific to the front and/or back lig-oligonucleotides;
a hybridization buffer;
a ligase;
a ligase buffer; and
a primer pair for adding phosphate groups to cDNA.

Another important aspect of the oligonucleotide dimer composition for selective amplification of single stranded cDNA described herein is the ability to select cDNA having known adapter sequences. This aspect could be applied for single cell sequencing where adapters are necessary for assigning reads to individual cells. In this application, cDNA sequences without cell identifying barcodes/adapters arise within the cDNA library. These are known as template switching oligo (TSO) artefacts and are undesirable in single cell sequencing projects due to not being assignable to a cell of origin. The present method can be applied to only select for cDNA sequences with the desired adapter sequences thus effectively limiting the sequencing of TSO artefacts. The present method can also be performed with single cell cDNA libraries to both remove TSO artefacts and to improve transcriptome coverage per cell.

TSO clean-up can also be carried out without normalization in which case it is not necessary to include a step of re-associating the cDNA sample to produce a mixture of post-association single stranded cDNA templates and post-association double stranded cDNA templates. Described herein is a method for diagnosing a disease in a subject, the method comprising:
(i) providing a cDNA sample from the subject comprising double stranded cDNA templates, a portion of the templates having a known 5' pre-attached adapter and a known 3' pre-attached adapter;
(ii) denaturing the cDNA sample to produce single stranded cDNA templates;
(iii) annealing a 5' adapter complex to the 5' pre-attached adapter of at least one single stranded cDNA template, and annealing a 3' adapter complex to the 3' pre-attached adapter of the same single stranded cDNA template, wherein each adapter complex comprises at least one oligonucleotide;
(iv) ligating an oligonucleotide from the 5' adapter complex to the 5' pre-attached adapter of the single stranded cDNA template and ligating an oligonucleotide from the 3' adapter complex to the 3' pre-attached adapter of the same single stranded cDNA template;
(v) selectively amplifying the cDNA sample using primers specific to the ligated oligonucleotides; and
(vi) sequencing the cDNA sample, wherein the sequencing output is used to identify whether the subject has the disease.

Described herein is a method for discovering a disease biomarker, the method comprising:
(i) providing a first cDNA sample from a subject with a disease and a second cDNA sample from a subject without the disease, the samples comprising double stranded cDNA templates, a portion of the templates having a known 5' pre-attached adapter and a known 3' pre-attached adapter;
(ii) denaturing each cDNA sample to produce single stranded cDNA templates;
(iii) annealing a 5' adapter complex to the 5' pre-attached adapter of at least one single stranded cDNA template, and annealing a 3' adapter complex to the 3' pre-attached adapter of the same single stranded cDNA template, wherein each adapter complex comprises at least one oligonucleotide;
(iv) ligating an oligonucleotide from the 5' adapter complex to the 5' pre-attached adapter of the single stranded cDNA template and ligating an oligonucleotide from the 3' adapter complex to the 3' pre-attached adapter of the same single stranded cDNA template;
(v) selectively amplifying each cDNA sample using primers specific to the ligated oligonucleotides; and
(vi) sequencing each cDNA sample and comparing the sequencing output for the first and second cDNA samples to discover a disease biomarker.

According to all aspects of the invention the cDNA sample may be from a single cell.

The embodiments of the method of selective amplification of single stranded cDNA recited above apply *mutatis mutandis* to the method of selective amplification of cDNA comprising known adapter sequences and are not repeated for reasons of conciseness. The oligonucleotide dimer composition is suitable for use in a method as defined herein for selective amplification of cDNA comprising known adapter sequences. The method of selective amplification of cDNA comprising known adapter sequences as defined herein can be used for discovery of a disease biomarker and/or a cancer vaccine target or in a process of diagnosing and/or prognosing a disease. The kits and sets of reagents defined herein are also suitable for use in the method for selective amplification of cDNA comprising known adapter sequences.

In some embodiments, according to all aspects of the invention, the cDNA sample comprises no more than 800ng, 700ng, 500ng, 100ng, 20ng, 10ng, 5ng or 1ng of starting cDNA. The cDNA sample may comprise 1-800 ng, 1-500ng, 5-100ng, or 10-50ng of starting cDNA.

In particular embodiments, according to all aspects of the invention, RNA from a sample is firstly reverse transcribed to cDNA. Sample types include blood samples (in particular from plasma, and also serum), other bodily fluids such as saliva, urine or lymph fluid. Other sample types include solid tissues, including frozen tissue or formalin fixed, paraffin embedded (FFPE) material. The RNA may be messenger RNA (mRNA), microRNA (miRNA) etc. In such embodiments, the RNA is typically reverse transcribed using a reverse transcriptase enzyme to form a complementary DNA (cDNA) molecule. Methods for reverse transcribing RNA to cDNA using a reverse transcriptase are well-known in the art. Any suitable reverse transcriptase can be used, examples of suitable reverse transcriptases being widely available in the art. The initial cDNA molecule may be single stranded until DNA polymerase has been used to generate the complementary strand. Commercially available kits (such as NEBNext ^{®} Single Cell/Low Input cDNA Synthesis & Amplification Module) can be used to convert RNA into double stranded cDNA with 5' and 3' adapters. Primers based on the 5' and 3' adapters can be used to add phosphate groups to the cDNA. A cDNA purification step (for example with ProNex or Ampure beads) may be carried out prior to use of the cDNA as a cDNA sample.

As the present invention only requires a low amount of starting cDNA, this can be produced from a small quantity of RNA and/or without the requirement for additional PCR cycles during the generation of the cDNA. The RNA sample may comprise no more than 3.5µg, 3µg, 2µg, 1µg, 500ng, 100 ng, 10ng or 1ng of starting RNA. The RNA sample may comprise 1ng-3µg, 10ng-2µg, or 100ng-1µg of starting RNA.

The invention also provides a system for discovering a disease biomarker, comprising:
(a) one or more testing devices for normalizing a first cDNA sample from a subject with a disease and a second cDNA sample from a subject without the disease and sequencing the normalized first and second cDNA samples;
(b) a processor; and
(c) storage medium comprising a computer application that, when executed by the processor, is configured to:
   (i) access the determined sequence(s) for the first and second cDNA samples on the one or more testing devices;
   (ii) calculate whether there is a cDNA sequence that is present in the first cDNA sample but not in the second cDNA sample or is present in the second cDNA sample but not in the first cDNA sample; and
   (iii) output from the processor the result of step (ii);
   wherein the one or more testing devices comprises an oligonucleotide dimer composition for selective amplification of single stranded cDNA by ligation of an oligonucleotide to a 5' and a 3' end of a single stranded cDNA template having known 5' and 3' pre-attached adapters, wherein the oligonucleotide dimer composition comprises:
   (A) a front oligonucleotide dimer comprising:
      (i) a front lig-oligonucleotide for ligating to the 5' pre-attached adapter of the post-association single stranded cDNA template; and
      (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
      such that, on annealing, an end of the front lig-oligonucleotide is adjacent an end of the 5' pre-attached adapter to enable ligation of the front lig-oligonucleotide to the 5' pre-attached adapter at a ligation site; and
   (B) a back oligonucleotide dimer comprising:
      (i) a back lig-oligonucleotide for ligating to the 3' pre-attached adapter of the post-association single stranded cDNA template; and
      (ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
      such that, on annealing, an end of the back lig-oligonucleotide is adjacent an end of the 3' pre-attached adapter to enable ligation of the back lig-oligonucleotide to the 3' pre-attached adapter at a ligation site.

Described herein is a system for discovering a cancer vaccine target, comprising:
(a) one or more testing devices for normalizing a first cDNA sample from a subject with cancer and a second cDNA sample from a subject without the cancer and sequencing the normalized first and second cDNA samples;
(b) a processor; and
(c) storage medium comprising a computer application that, when executed by the processor, is configured to:
   (i) access the determined sequence(s) for the first and second cDNA samples on the one or more testing devices;
   (ii) calculate whether there is a cDNA sequence that is present in the first cDNA sample but not in the second cDNA sample or is present in the second cDNA sample but not in the first cDNA sample; and
   (iii) output from the processor the result of step (ii).

In a related aspect, there is provided a system for diagnosing a disease in a subject, comprising:
(a) one or more testing devices for normalizing a cDNA sample from a subject and sequencing the normalized cDNA sample
(b) a processor; and
(c) storage medium comprising a computer application that, when executed by the processor, is configured to:
   (i) access the determined sequence(s) for the cDNA sample on the one or more testing devices
   (ii) calculate whether a cDNA sequence is present or absent, wherein the presence or absence of the cDNA sequence is associated with the disease; and
   (iii) output from the processor whether a subject has the disease;
   wherein the one or more testing devices comprises an oligonucleotide dimer composition for selective amplification of single stranded cDNA by ligation of an oligonucleotide to a 5' and a 3' end of a single stranded cDNA template having known 5' and 3' pre-attached adapters, wherein the oligonucleotide dimer composition comprises:
   (A) a front oligonucleotide dimer comprising:
      (i) a front lig-oligonucleotide for ligating to the 5' pre-attached adapter of the post-association single stranded cDNA template; and
      (ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
      such that, on annealing, an end of the front lig-oligonucleotide is adjacent an end of the 5' pre-attached adapter to enable ligation of the front lig-oligonucleotide to the 5' pre-attached adapter at a ligation site; and
   (B) a back oligonucleotide dimer comprising:
      (i) a back lig-oligonucleotide for ligating to the 3' pre-attached adapter of the post-association single stranded cDNA template; and
      (ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
      such that, on annealing, an end of the back lig-oligonucleotide is adjacent an end of the 3' pre-attached adapter to enable ligation of the back lig-oligonucleotide to the 3' pre-attached adapter at a ligation site.

Described herein is a system for prognosing a disease in a subject, comprising:
(a) one or more testing devices for normalizing a cDNA sample from a subject and sequencing the normalized cDNA sample
(b) a processor; and
(c) storage medium comprising a computer application that, when executed by the processor, is configured to:
   (i) access the determined sequence(s) for the cDNA sample on the one or more testing devices
   (ii) calculate whether a cDNA sequence is present or absent, wherein the presence or absence of the cDNA sequence is associated with the disease; and
   (iii) output from the processor a prognosis for the disease.

The one or more testing devices may use/comprise an oligonucleotide dimer composition as described herein. The one or more testing devices may comprise a long-read sequencer.

The system may further comprise a display for the output from the processor.

There is also described herein a computer application or storage medium comprising a computer application as defined herein.

### DETAILED DESCRIPTION

The above and other aspects of the present invention will now be described in further detail, by way of example only, with reference to the following examples and the accompanying figures, in which:
Figure 1 is a schematic overview showing addition of ligation sequences to the ends of single stranded cDNA templates;
Figure 2 is a schematic overview of an embodiment of a cDNA normalization process in accordance with the present invention;
Figure 3 is a schematic overview of Front and Back oligonucleotide dimer structures as shown annealed to single strand cDNA template, Fig 3A is a detail thereof and Fig 3B provides an illustration using the Example sequence representations recited herein;
Figure 4 is a graph showing length distribution from gel electrophoresis of input cDNA;
Figure 5 is a graph showing length distribution from gel electrophoresis of normalized cDNA resulting from a cDNA normalisation process as shown in Figure 2; and
Figure 6 shows saturation curves for input cDNA and a normalized cDNA normalized as in Figure 2 using Nanopore cDNA sequencing.
Figure 7 is a 2D PCA-plot from 13573 transcripts showing clustering of cancer and control samples in the dataset.

To address issues in current cDNA normalization technology, the present inventors have developed an improved selective amplification method (termed "Level-Up"). This method utilises the same denaturation and re-hybridization process as the DSN and column methods described herein. However, the present method differs from other approaches by using a non-depletion or additive mechanism. In other words, Level-Up provides a method and means for increasing the amount of low abundance cDNA in a sample. These methods and means can be used for cDNA normalization in sequencing processes or for other processes that would benefit from amplification of low abundance cDNA, such as discovery, detection or identification of biomarkers.

In the context of the present invention, the following explanations of terms and methods are provided to better describe the present disclosure and to provide guidance in the practice of the present disclosure.

The phrase 'selective amplification' is used to describe the method developed by the present inventors of amplification of particular DNA templates in preference to other DNA templates, for example, amplification of only single stranded cDNA in a sample that comprises a mixture of single stranded and double stranded cDNA. The term is also used herein to describe preferentially amplifying a particular category of DNA, such as low abundance DNA.

The term 'adapter' is used to describe a short DNA sequence added to an end of a DNA template, such as those commonly used in RNA sequencing by ligating an adapter to a cDNA template. A '3' pre-attached adapter' refers to an adapter having a known nucleotide sequence that has been added to the 3' end of a cDNA template. A '5' pre-attached adapter' refers to an adapter having a known nucleotide sequence that has been added to the 5' end of a cDNA template.

The terms 'normalized' and 'normalized fraction' refer to the process of levelling the abundance of different transcripts within a sample. This can be achieved by prior art methods of reducing the amount of highly abundant transcripts, or by using the methods described herein to selectively amplify low abundance transcripts.

The phrase 'post-association single stranded cDNA template' as used in the context of the present invention, refers to a single stranded cDNA template that has been generated by disassociating and re-associating (i.e., denaturing and re-hybridizing) a sample of double stranded cDNA to form a mixture of single stranded cDNA and double stranded cDNA. The single stranded cDNA that remains single stranded after re-association is referred to as post-association single stranded cDNA. If a re-association step is not carried out, the phrase 'post-association single stranded cDNA template' is interchangeable with 'single stranded cDNA template' in the embodiments defined herein.

The phrase 'ligated-adapter-cDNA template' as used herein, refers to a cDNA template formed by ligation of an adapter to a cDNA template.

The present invention encompasses an 'adapter complex' which is suitable for annealing to an end of a post-association single stranded cDNA template. The term 'adapter complex' refers to an adapter that comprises more than one component.

The terms 'front oligonucleotide dimer', 'front k-linker' and 'front dimer' as used in the context of the present invention, refer to an adapter complex that can be annealed to the 5' end of a post-association single stranded cDNA template. The terms 'back oligonucleotide dimer', 'back k-linker' and 'back dimer', as used in the context of the present invention, refer to an adapter complex that can be annealed to the 3' end of a post-association single stranded cDNA template.

The terms 'front lig-oligonucleotide' and 'front lig' as used in the context of the present invention, refer to an oligonucleotide component of the front dimer. The terms 'back lig-oligonucleotide' or 'back lig' as used in the context of the present invention, refer to an oligonucleotide component of the back dimer.

The terms 'front link-oligonucleotide' and 'front link' as used in the context of the present invention, refer to an oligonucleotide component of the front dimer. The terms 'back link-oligonucleotide' and 'back link' as used in the context of the present invention, refer to an oligonucleotide component of the back dimer.

The term 'overhang' is used in the context of the present invention to describe an overhanging region of the sequence of the dimer of the present invention, where the overhanging region is non-complementary to the region with which it is paired, once annealed to a post-association single stranded cDNA template, such that the overhanging region does not bind with its paired region.

The single stranded cDNA is selectively amplified using primers specific to the ligated oligonucleotides. As single stranded DNA is the template, the primer region of one primer of a specific primer pair is complementary to the single stranded DNA molecule. The other primer of the specific primer pair comprises a primer region which is complementary to, and therefore hybridises with, the complementary single stranded DNA molecule formed during an amplification cycle. Thus, one primer is complementary to one of the ligated oligonucleotides and the other primer comprises (at least partially) the sequence of the other ligated oligonucleotide.

### Previously developed methods of cDNA normalization

There are two forms of full length cDNA normalization that have been previously developed: the Duplex Specific Nuclease (DSN) method (Zhulidov, P. A. et al. Simple cDNA normalization using kamchatka crab duplex-specific nuclease. Nucleic Acids Res. 32, e37 (2004)) and the hydroxyapatite column method (Andrews-Pfannkoch, C., Fadrosh, D. W., Thorpe, J. & Williamson, S. J. Hydroxyapatite-mediated separation of double-stranded DNA, single-stranded DNA, and RNA genomes from natural viral assemblages. Appl. Environ. Microbiol. 76, 5039-5045 (2010)). Both methods rely on the denaturation and re-hybridization of cDNA strands. As the single stranded cDNA move about in solution, the sequences that are more highly abundant have a greater probability of finding a matching complementary sequence with which to re-hybridize. Thus, as re-hybridization reaches its limit, the remaining single stranded cDNA represents a normalized sequence library.

The difference between the two methods lies in their approach for isolating the single stranded cDNA library from the re-hybridized double stranded cDNA molecules.

In the DSN method, an enzyme which specifically cleaves double stranded DNA is used to decompose all double stranded cDNA within the solution. The solution is then purified and size-selected for cDNA sequences above a certain length. These sequences are then amplified using the Polymerase Chain Reaction (PCR).

In the column method, the denatured and re-hybridized cDNA library is passed through a heated column filled with hydroxyapatite granules. The hydroxyapatite preferentially binds to larger DNA molecules. The size of DNA that is bound is controlled by the concentration of phosphate buffer in which the cDNA library is dissolved. Thus the concentration of phosphate buffer must be tuned specifically for cDNA molecules within a certain range of sequence length. The cDNA is eluted through the column using increasing concentrations of phosphate buffer to extract increasing sizes of DNA molecules. Since the single stranded cDNA will be roughly one half the size of the re-hybridized cDNA, elution of the single stranded fraction can be managed if the mean cDNA sequence length is known. The resulting elution is intended to be enriched for the single stranded cDNA which are then amplified using PCR.

In both the DSN and column methods, known adapters must be attached to the ends of the cDNA prior to normalization to facilitate PCR amplification (so that appropriate primers can be used).

Since both methods are subtractive by nature with the depletion of large fractions of cDNA, the amount of starting cDNA is typically required to be higher than 1µg for the DSN approach and 4µg for the column approach.

Since the DSN method uses enzymes which cleave all double stranded cDNA, in theory it can deplete low abundance sequences with segments that match high abundance sequences. This effect can also increase the probability of forming PCR chimeras. PCR chimeras are formed when incomplete single stranded cDNA sequences act as primers to other sequences thus combining the sequences in a way that does not occur in nature. PCR chimeras represent false positives for novel isoforms and are extremely challenging to distinguish from true alternative isoforms. Validating PCR chimeras typically requires in-depth biochemical assays.

Since the column method only allows for segregation of high abundance and low abundance fractions within a narrow size range, it has significant bias against longer cDNA sequences. The effect of this is a loss of representation for longer RNA sequences.

### Column method

As described above the hydroxyapatite column method relies on the denaturation and re-hybridization of cDNA strands. As the single stranded cDNA move about in solution, the sequences that are more highly abundant have a greater probability of finding a matching complementary sequence with which to re-hybridize. In the column method, the denatured and re-hybridized cDNA library is passed through a heated column filled with hydroxyapatite granules. The hydroxyapatite preferentially binds to larger DNA molecules. Since the single stranded cDNA will be roughly one half the size of the re-hybridized cDNA, elution of the single stranded fraction can be managed if the mean cDNA sequence length is known. The resulting elution is intended to be enriched for the single stranded cDNA which are then amplified using PCR.

The hydroxyapatite column method was carried out as described in Andrews-Pfannkoch et al. (Andrews-Pfannkoch, C., Fadrosh, D. W., Thorpe, J. & Williamson, S. J. Hydroxyapatite-mediated separation of double-stranded DNA, single-stranded DNA, and RNA genomes from natural viral assemblages. Appl. Environ. Microbiol. 76, 5039-5045 (2010) with 4µg cDNA starting sample. The hydroxyapatite column method did not produce usable yield when used with 2µg or less of cDNA.

As the column method is based on separation by size this method results in a loss of representation for longer RNA sequences (longer than 4kb) which is observable in the length distribution before and after normalization.

### DSN method

As for the column method, the DSN method relies on the denaturation and re-hybridization of cDNA strands. As the single stranded cDNA move about in solution, the sequences that are more highly abundant have a greater probability of finding a matching complementary sequence with which to re-hybridize. In the DSN method, an enzyme which specifically cleaves double stranded DNA is used to decompose all double stranded cDNA within the solution.

The commercially available Evrogen Trimmer-2 cDNA normalization kit uses the DSN method. This kit was used according to the manufacturer's instructions with 1µg cDNA starting sample. However, to produce enough material for long read RNA sequencing it was found necessary to use 2µg of cDNA.

The DSN method was found to completely eradicate high abundance RNAs and this would also be expected to be the case for RNAs with sequence similarity to the high abundance RNAs. Thus, over-depletion was observed in which high abundance RNAs were not just reduced in quantity but were completely removed from the samples. Table 1 illustrates over-depletion of ranks 1-20 and shows a selection of lower ranks (55, 64, 77, 92 and 98) in which the RNAs were significantly reduced but not completely depleted.

**Table 1 - over-depletion of high abundance RNAs with DSN method**

| Rank | Ensembl Transcript ID | Standard Read Count | DSNase Read Count |
|---|---|---|---|
| 1 | ENSGALT00000023323 | 18937 | 0 |
| 2 | ENSGALT00000039524 | 14928 | 0 |
| 3 | ENSGALT00000052417 | 9147 | 0 |
| 4 | ENSGALT00000022187 | 4293 | 0 |
| 5 | ENSGALT00000011687 | 3822 | 0 |
| 6 | ENSGALT00000041222 | 3610 | 0 |
| 7 | ENSGALT00000016648 | 2996 | 0 |
| 8 | ENSGALT00000044106 | 2949 | 0 |
| 9 | ENSGALT00000084298 | 2825 | 0 |
| 10 | ENSGALT00000023065 | 2677 | 0 |
| 11 | ENSGALT00000088434 | 2255 | 0 |
| 12 | ENSGALT00000011524 | 2151 | 0 |
| 13 | ENSGALT00000055441 | 2127 | 0 |
| 14 | ENSGALT00000044027 | 2080 | 0 |
| 15 | ENSGALT00000078059 | 2052 | 0 |
| 16 | ENSGALT00000084500 | 2020 | 0 |
| 17 | ENSGALT00000071026 | 1839 | 0 |
| 18 | ENSGALT00000015018 | 1742 | 0 |
| 19 | ENSGALT00000046070 | 1731 | 0 |
| 20 | ENSGALT00000014652 | 1718 | 0 |
| 55 | ENSGALT00000066490 | 649 | 1 |
| 64 | ENSGALT00000087166 | 630 | 5 |
| 77 | ENSGALT00000072914 | 565 | 1 |
| 92 | ENSGALT00000043160 | 475 | 1 |
| 98 | ENSGALT00000070660 | 451 | 2 |

In addition, the DSN method creates conditions in which artificial chimeric sequences can be generated, which can show up as false positives for gene predictions.

### Selective amplification method (Level-up)

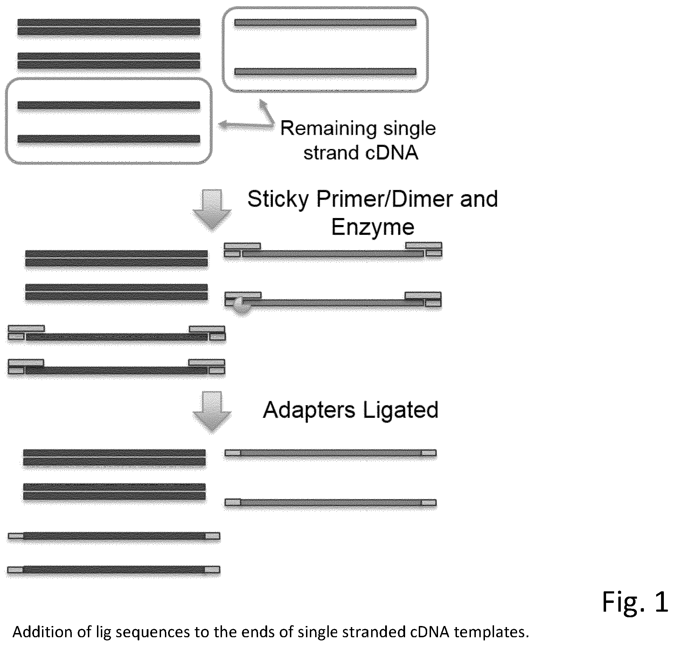

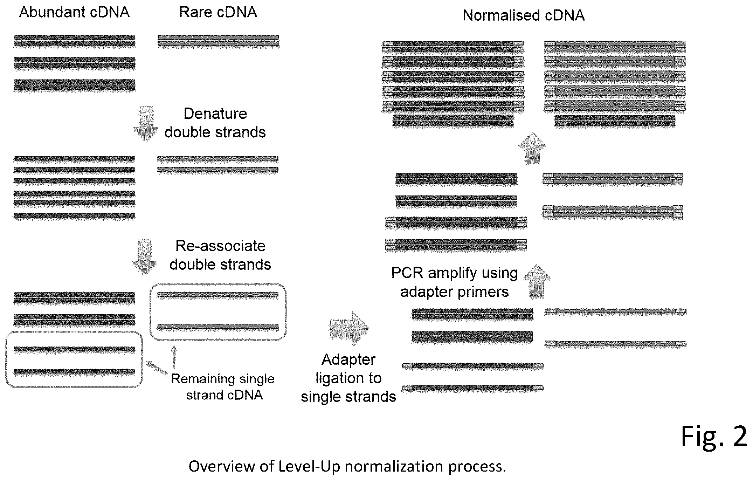

This method is illustrated in Figures 1 to 3. In overview, with reference to Figure 2, a cDNA sample, comprising cDNA having known 5' and 3' pre-attached adapters, is denatured to provide a single stranded cDNA sample; the sample is then re-hybridised or re-associated to provide a mixture of single and double stranded cDNA. This single stranded cDNA represents the low abundance cDNA. The single stranded cDNA within the re-associated sample is then modified by adding oligonucleotides to the 5' and 3' pre-attached adapters. The cDNA sample is amplified using primers to these oligonucleotides. This process selectively increases the content of only the single stranded cDNA within the re-associated sample, thereby increasing the content of the low abundance cDNA in the sample, thereby providing a normalized sample.

The input cDNA library for the selective amplification method is double-stranded and the double stranded templates each comprise a 5' pre-attached adapter of known nucleotide sequence and a 3' pre-attached adapter of known nucleotide sequence. Since the present method is an additive method, lower quantities of starting cDNA are required as compared to prior art normalization methods. In the DSNase method, a minimum of 1µg of input cDNA is required and in the column method a minimum of 4µg of input cDNA is required. In testing, it was found that the present method could be applied with as little as 20ng of starting cDNA.

The input cDNA is combined with a hybridization buffer and the solution heated to denaturation temperatures - which is about 98 degrees Celsius, to produce denatured single stranded cDNA templates. After 5-10 minutes, the solution is then brought down to re-hybridization temperature - about 68 degrees Celsius. The solution is incubated at this temperature for between 0-24 hours depending on the amount of normalization required, for example 3-10 hours. 7 hours is a typical duration for the re-association step. This step produces a re-associated or re-hybridised sample comprising post-association double stranded cDNA templates and post-association single stranded cDNA templates.

After incubation, oligonucleotide dimers (termed K-linkers by the present inventors), of the present invention are added. These oligonucleotide dimers are discussed in more detail below. At this point, the solution can be left to incubate at 68 degrees Celsius from 0-1 hour. 5 minutes is a typical duration for this incubation step. The solution is then brought down to the annealing temperatures of the K-linkers, which is typically between 40-60 degrees Celsius, for example 44 degrees celsius. The solution is incubated at the temperature from between 10 minutes-2 hours, for example 25 minutes. This step anneals the K-linkers to the post-association single stranded cDNA templates.

After this incubation period, DNA ligase is added together with ligation mix. The solution is incubated at this same temperature for 0.5-2 hours, for example 1 hour, and then brought down to room temperature. This step results in formation of ligated-adapter-cDNA templates where an oligonucleotide from a K-linker is ligated to each end of a post-association single stranded cDNA template. At this point, the cDNA may be purified (for example using Pronex or Ampure beads) or may be used directly for PCR amplification using primers based on the K-linker sequences.

After PCR amplification, the cDNA is then purified using any appropriate means and the resultant cDNA represents the normalized cDNA library.

The post-association double stranded cDNA templates can be removed before PCR amplification but, after testing, the present inventors have shown that leaving the double stranded cDNA in the solution does not negatively impact the normalization process.

Indeed the post-association double stranded cDNA can also be analysed and used, for example, to attain estimates of gene expression. This involves an additional (PCR) selective amplification step using primers to the known 5' pre-attached adapter and known 3' pre-attached adapter, wherein molecular barcodes are included in the primers. PCR amplification using primers based on the K-linker sequences is carried out first followed by a single PCR cycle using the primers to the known 5' pre-attached adapter and known 3' pre-attached adapter. The PCR can be paused to add the further primers for the final cycle. Alternatively, the cDNA can be purified after the PCR using the primers based on the K-linker sequences and a new PCR carried out for a single cycle using the primers to the known 5' pre-attached adapter and known 3' pre-attached adapter. In both cases the product will be a mixture of two distinguishable fractions comprised of sequences originating from the post-association double stranded cDNA templates and from the post-association single stranded cDNA templates. The molecular barcode addition allows for the identification of the source molecule during sequencing analysis. This aspect of the invention may be used in multiplex sequencing.

### Design of oligonucleotide dimer complexes

The oligonucleotide dimer compositions of the present invention comprise front and back oligonucleotide dimers (front and back K-linkers), which both anneal to the same strand of post-association single stranded cDNA.

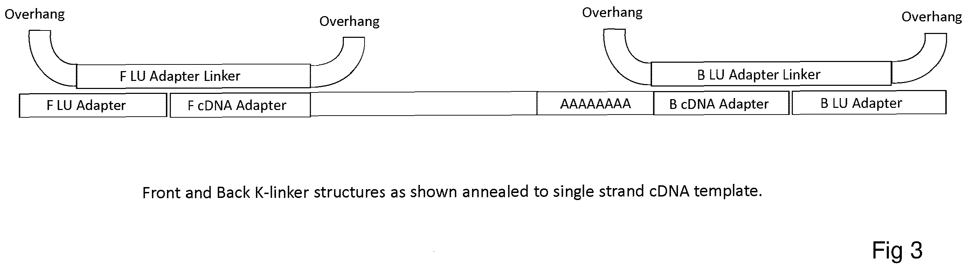

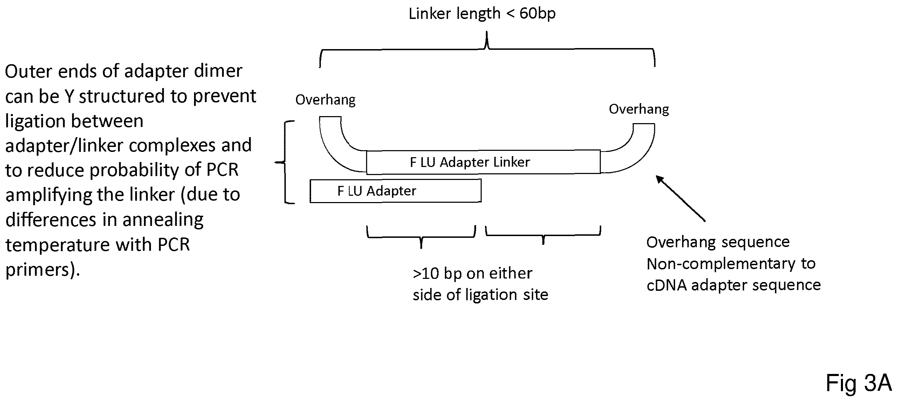

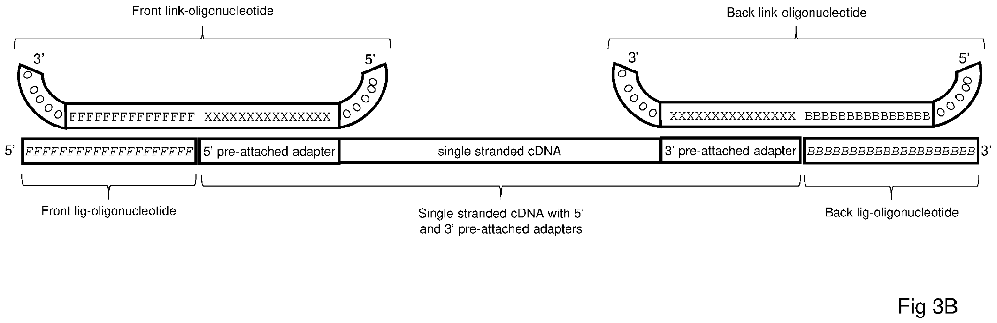

With reference to Figures 3 and 3A, each K-linker comprises two oligonucleotide sequences; one is termed the link-oligonucleotide (also termed 'link'; termed 'LU adapter linker' in Figures 3 and 3A)) and the other is termed the lig-oligonucleotide (also termed 'lig'; termed 'LU adapter' in Figures 3 and 3A).

The link sequence includes a region complementary with the known 3'/5' pre-attached adapter sequence (that was previously added to the cDNA) and a region complementary with the lig.

As also depicted in Figures 3 and 3A, the link can be designed to have an overhang region at one end which is non-complementary the known pre-attached adapter sequences, this region is termed a 'template overhang'. The opposite end of the link can be provided with a similar overhang that is non-complementary to the lig sequence, this is termed a 'lig overhang' or 'lig-oligonucleotide overhang'.

The purpose of the link is to anneal to both the pre-attached adapter of the post-association single stranded cDNA template and the lig, in such a way that, in use, one end of the cDNA template is adjacent one end of the lig. By positioning the cDNA template and the lig in this way, DNA ligase can be used to ligate the cDNA template to the lig, thus adding the lig sequence to the end of the cDNA template. A lig sequence is added to both the 5' and 3' ends of the single stranded cDNA template. Front and back K-linkers are used to add these ligs to the 5' and 3' ends of the cDNA template respectively.

In the method described above, once a lig has been added to each end of the single stranded cDNA template, primers based on the sequences of the added ligs are used to selectively amplify the single stranded cDNA fraction that has successfully ligated to both front and back lig sequences. In this way, PCR can be used to amplify only the low abundance post-association single stranded cDNA fraction.

The particular structure of the K-linkers provides advantages to overall normalization performance with the front K-linker and the back K-linker having different functions provided by their specific structural characteristics.

The front K-linker which binds to the 5' end of the single strand cDNA template (shown in the figures as the reverse complement to the original RNA sequence) can be designed so that the K-linker does not act as a primer during PCR amplification.

To provide additional advantages, the front K-linker does not have a blunt end on the lig side to allow for the use of DNA ligase that can perform blunt end ligation in the selective amplification process. Providing the lig side of the K-linker complex with a non-blunt end also avoids ligation to other K-linker complexes or to the double stranded cDNA in the solution.

Since the front linker has a 5' to 3' directionality pointing away from the template, the linker itself cannot act as a primer of the template. However, in some instances, the front lig or PCR primers could potentially anneal to the linker during PCR amplification and undergo polymerase extension to take on the template side sequence of the link. Providing the link with an overhang on the template side avoids the extended lig/primer acting as a primer for the cDNA sequences which do not have the lig sequences added to their ends, i.e., the sequences that are high abundance. Accordingly, a template overhang structure for the back link can be provided.

The back K-linker which binds to the 3' end of the single stranded cDNA template (shown in the figures as the reverse complement to the original RNA sequence) can be designed so that the K-linker does not act as a primer during PCR amplification. This can be achieved using the template overhang, which is described above and illustrated in Figures 3 and 3A.

The back K-linker can be provided without a blunt end on the lig side for the same reason that the front K-linker can be designed to have an overhang on the lig side. If the lig side of the back K-linker complex had a blunt end, in some instances, the lig could potentially be ligated to other K-linker complexes or to the double stranded cDNA in the sample, which could result in linking of cDNA templates.

The overhangs also serve to lower the annealing temperature of the K-linker complexes so that they are less likely to act as primers for each other during PCR amplification, where higher annealing temperatures are used. Overhangs may reduce unintended priming. Additionally or alternatively, overhangs may provide an indicator that enables the measurement of the amount of unintended priming. For example, if the K-linker complexes with their overhangs are able to prime a template then it will be possible to see the overhang sequence in the sequencing data and to conclude that it was a product of un-intended priming.

All overhangs should ideally be between about 1bp and about 20bp, preferably 3bp. Longer overhangs could be used but it would make the design more difficult since there are fewer sequence compositions that would prevent unintended priming as the overhangs get longer.

The complementary regions between the cDNA template and the link, and between the link and the lig should be long enough for annealing at the temperature of activity for the ligase to be used. Nick repair ligases are particularly preferred for this process which typically require about five or more complementary bases on either side of the ligation site.

The combined length of the link and corresponding lig should ideally be less than about 300bp, preferably less than about 200bp, to reduce carry over during the purification process.

### Example sequence representations:

All example sequence structures are provided in 5' to 3' orientation. Also illustrated in Figure 3B.
Front link:
   OOOOOXXXXXXXXXXXXXXXFFFFFFFFFFFFFFFOOOOO
Front lig:
   *FFFFFFFFFFFFFFFFFFFF*
Back link:
   OOOOOBBBBBBBBBBBBBBBXXXXXXXXXXXXXXXOOOOO
Back lig:
   *BBBBBBBBBBBBBBBBBBBB*
X - Nucleotides complementary to 5' / 3" pre-attached adapter
O - Overhang sequences
F - Sequence complementary to front lig to be ligated
*F -* Sequence of the front lig
B - Sequence complementary to back lig to be ligated
*B* - Sequence of the back lig

### Example of oligonucleotides and single stranded cDNA template used in the selective amplification method:

Primer sequences from NEB/PacBio cDNA synthesis kit
Iso-Seq Express Fwd:
   GGCAATGAAGTCGCAGGGTTG
Iso-Seq Express Rev:
   AAGCAGTGGTATCAACGCAGAG
Front Link:
   ATAGCGTTGATACCACTGCTT**CTCACGACAGACTCGC**TAA
Front Lig and Primer:
   TGGACTGAT ***GCGAGTCTGTCGTGAG***
Back Link:
   AAT**GACGCTGGACGAACAC** GGCAATGAAGTCGCAGACA
Back Lig:
   ***GTGTTCGTCCAGCGTC*** CAGGTGAGTGG
Primer:
   CCACTCACCTG **GACGCTGGACGAACAC**

Overhangs are shown underlined. Regions of complementarity between oligonucleotides are shown in bold.

Single stranded cDNA template:
**AAGCAGTGGTATCAACGC**AGAGNNNNNNNNNNNNNNNNCAACC**CTGCGACTTCATT GCC** (i.e. 5'-3' - sequence of 5' pre-attached adapter, sequence of cDNA represented by N and sequence of 3' pre-attached adapter; regions of complementarity to front link and back link-oligonucleotides are shown in bold).

### Preparation of oligonucleotide dimers

Once designed, the dimers can be prepared using standard techniques well known in the art.

### Amplification of oligonucleotide-cDNA templates

After ligation of the lig to the 3' and 5' ends of the cDNA template, the resulting solution can be purified for cDNA using an appropriate cDNA purification method. The purification step may be skipped, but skipping may result in lower efficiency for PCR amplification.

After purification or after ligation the resulting material can be PCR amplified using forward and reverse primers based on the lig sequence. The primer sequences can be chosen to have a higher annealing temperature than the complementary regions of the template/link/lig to avoid unwanted priming.

If required, the number of optimal PCR cycles can be identified by first running a qPCR experiment to identify the inflection point of the amplification curve.

The resulting cDNA from PCR amplification can then be purified and used as input for any downstream processes, such as sequencing.

### Validation of the selectively amplified sample

The effect of selective amplification or normalization can be indirectly measured by measuring the length distribution of the cDNA library using gel electrophoresis or directly measured using sequencing.

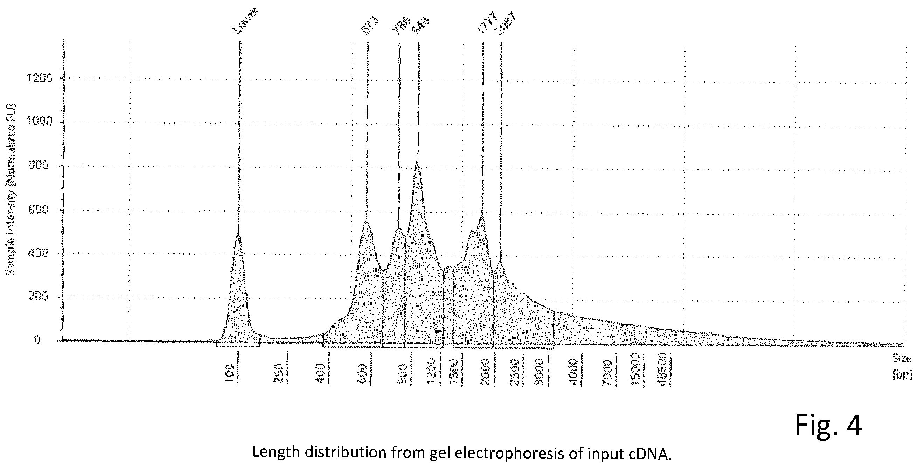

To identify the effect of normalization using gel electrophoresis, the length distribution plots of the input cDNA can be compared to the normalized cDNA. The input cDNA will typically show peaks along the length distribution which correspond to high abundance transcript sequences (Figure 4).

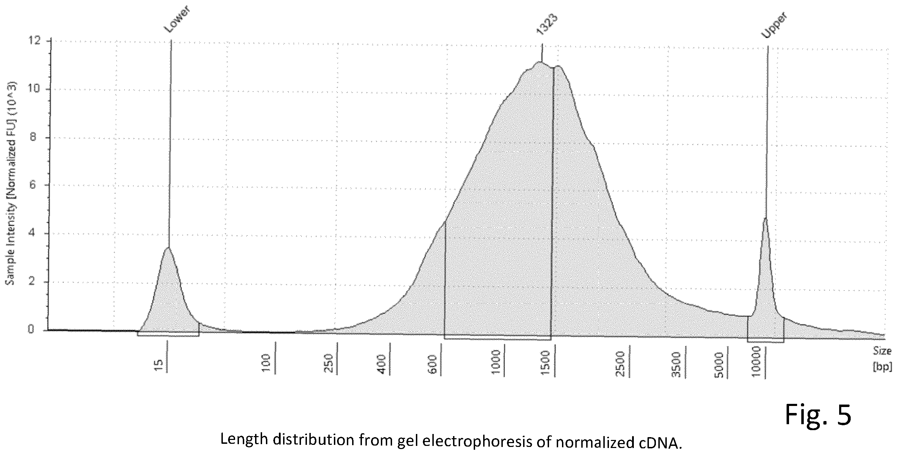

The normalized cDNA will have a length distribution that resembles a normal distribution with no sharp peaks (Figure 5). This represents a uniform distribution across transcript sequences.

When using sequencing for direct measurement of normalization, the preferred method of sequencing is long read sequencing. This allows for the identification of distinct isoforms. The results for direct measurement can be either a plot of the number of reads per gene or a saturation plot showing the number of new genes identified with increase depth of sequencing.

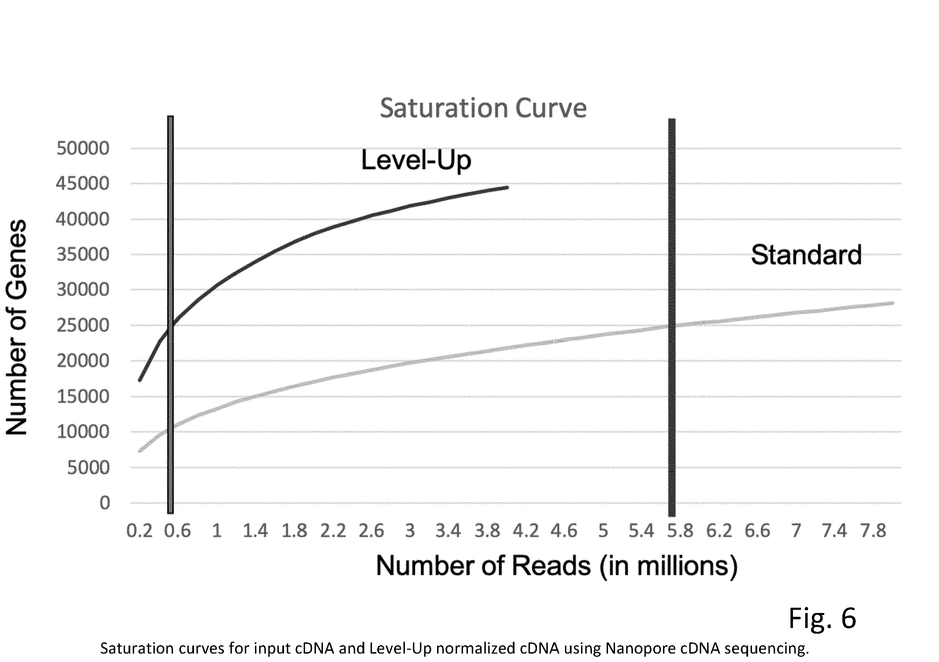

To validate the present method, the inventors performed Nanopore cDNA sequencing on both the input cDNA library and a cDNA library generated by the present method. They compared the saturation curves for each of the libraries (Figure 6). The curve for the present method is higher than the curve for the input cDNA by multiple factors. This indicates a more uniformly distributed cDNA library.

### Applications of selective amplification

A primary application for the present method of selective amplification is for improving the discovery and detection of low abundance genes and isoforms. When combining the present method with sequencing, the sampling efficiency is increased for identifying all unique genes within a sample.

The present method can be applied to any double stranded cDNA library with known adapters on the ends and lengths that can be amplified via the PCR method. This means that it can be used in DNA sequencing.

Another important aspect of the present method is the ability to select cDNA having known lig sequences. This aspect could be applied for single cell sequencing where adapters are necessary for assigning reads to individual cells. In this application, cDNA sequences without cell identifying barcodes/adapters arise within the cDNA library. These are known as template switching oligo (TSO) artefacts and are undesirable in single cell sequencing projects due to not being assignable to a cell of origin. The present method can be applied with a short re-hybridization step to only select for cDNA sequences with the desired lig sequences thus effectively limiting the sequencing of TSO artefacts. The present method can also be performed with single cell cDNA libraries to both remove TSO artefacts and to improve transcriptome coverage per cell.

### Discussion

The present method of selective cDNA amplification represents an innovative method of achieving greater non-targeted discovery/detection of low abundance nucleic acids. Being non-targeted it is not necessary to know which sequences are of low or high abundance in a given sample.

It differs from existing normalization approaches by using an additive method where the current approaches use a depletion method. The additive method allows for the present method to be used with significantly smaller amounts of starting cDNA. It aids the detection of low abundance nucleic acids and provides for greater confidence in determining the absence of particular nucleic acids. The additive method also prevents over depletion and artificial chimerization, which is endemic to the DSNase method. The present method is also only length biased to the degree that PCR amplification is length biased. Thus it can be run successfully with longer cDNA molecules.

Alternative embodiments are included in the examples described herein.

Although specific embodiments have been described above in detail, the description is merely for purposes of illustration. It should be appreciated, therefore, that many aspects described above are not intended as required or essential elements unless explicitly stated otherwise.

### EXAMPLES

The present invention will be further understood by reference to the following experimental examples.

### EXAMPLE 1

### Using a novel transcriptomic discovery platform to detect unique RNA signatures in epithelial cancers

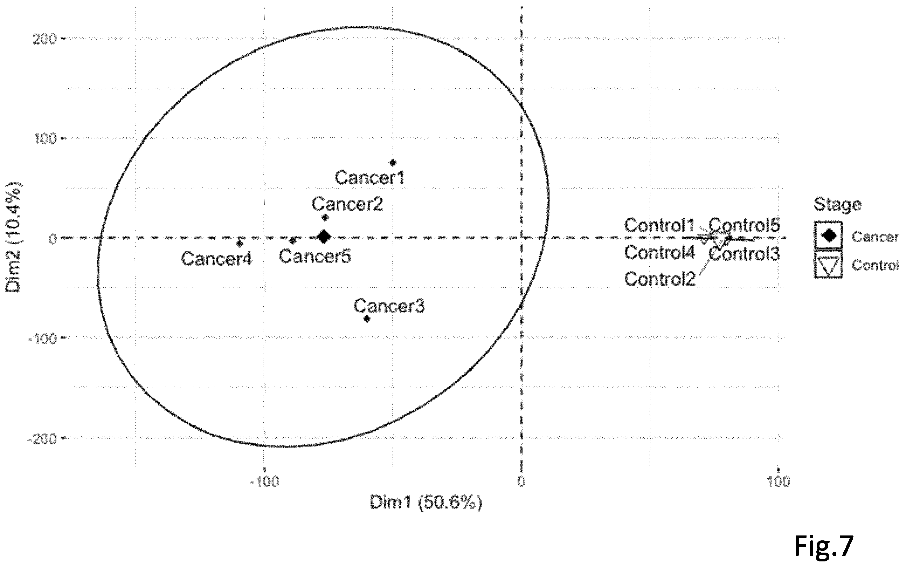

The present inventors have validated the use of Level-up with RNA from cancer cell lines and blood samples. In these initial studies thousands of previously un-annotated isoforms which have the potential to be used as cancer biomarkers have been found. Figure 7 shows the clustering of cancer samples (blood samples taken from people who have been diagnosed with breast cancer) and control samples (blood samples taken from people who have not been diagnosed with breast cancer) based on 13573 transcripts identified in these initial studies. The cancer samples can be readily distinguished from the control samples.

An example protocol is provided for discovery of unique RNA signatures in epithelial cancers:

### Stage 1

Blood handling validation is performed using 5 samples from 5 individuals (25 total samples). Blood is collected and stored in PAXgene RNA blood tubes (each with a blood capacity of 2.5 ml). After collection the tubes are transported on dry ice the same day from the local collection centre.

For each biological replicate the 5 collected tubes are processed according to different simulated handling situations. These include:
1. Processing tube on the same day as collection.
2. Processing tube 72 hours after collection.
3. Processing tube 2 weeks after collection.
4. Processing tube 4 weeks after collection.
5. Storing tube long term at -80C to be processed in 4-12 months.

RNA is extracted from the PAXgene tubes using a Qiagen PAXgene Blood RNA Kit (Cat. No. / ID: 762164). The resulting RNA is converted into cDNA using the NEBNext^{®} Single Cell/Low Input cDNA Synthesis & Amplification Module (E6421S). The resulting cDNA is processed by normalization as described herein. The normalized cDNA library will then be sequenced using an Oxford Nanopore Technologies Minion MK1C sequencer.

### Stage 2

Having optimised sample processing and assured quality of data generated, the study then progresses to patient samples.

### Inclusion and exclusion criteria

Inclusion criteria: All female patients who are pre-menopausal, over 18 years of age but under 50 years with invasive breast cancer or a benign condition (B2).

Exclusion criteria: History of any cancer or a concurrent cancer of other type, auto-immune diseases, and women not able or willing to give informed consent.

### Identification and recruitment of patients to the prospective cohort study

Eligible patients are identified from multidisciplinary team (MDT) meetings, with the results of triple assessment. When patients attend for results at the clinic, with the breast surgeon, they are invited to participate in the study and given a patient information sheet (PIS). Patients given a PIS will be followed up by the research team after 72 hrs. If the patient elects to participate in the study, they meet with the research nurse and sign a consent form or be offered to be consented remotely.

### Anonymisation

Once enrolled in the study, the research nurse assigns the patient with a unique ID number that is linked through a secure database to the background data and used therein to collect and identify samples.

### Background data collection

The following data is collected for each patient: Baseline demographic, menopausal status, past medical history, medication history, drug and alcohol consumption, BMI and family history.

Triple assessment including presentation, examination findings, imaging, and biopsy results is collated. Post-surgical histology is also be collected. Any additional prognostic information, such as use of biomolecular assays is also collected.

### Blood sample collection

On the morning of surgery, a blood sample is obtained and stored at -20°C to be transported to the processing centre. For any patient who is not proceeding to surgery e.g. fibroadenoma, they are invited to attend the day unit where blood tests are taken by the same team. The sample size is 20ml with a maximum collection that day of 30ml.

### Processing of blood samples

Blood samples are processed from the collection tubes in a laminar flow class 2 safety cabinet using an RNA extraction kit provided by Qiagen. After RNA has been extracted, the remaining solid waste is autoclaved and discarded using a supplier of human medical waste removal services. Liquid waste will be decontaminated and disposed as per University of Edinburgh Guidance on use of human samples. Blood samples that are not processed on the same day of arrival are stored in a secure -80°C freezer. A sealed container is used to transport blood samples between the freezer and the safety cabinet.

### Data analysis

Transcriptomics data is stored and processed in secure Amazon Web Services cloud repositories and servers. There is no personal information stored in the same computational location.

Oxford Nanopore Technologies (ONT) Minion sequencing machines are used to run cDNA sequencing on the samples. This outputs raw data as fast5 files. The most up-to-date high accuracy basecaller from ONT (Bonito) is run to convert to fastq sequence files. The nanopore reads are filtered for quality using seqkit and adapters removed using pychopper. The trimmed reads are then mapped to the HG38 (or newer version) human reference genome assembly using Minimap2.

### Stages 3-4

The study is amended to initially increase the sample size and subsequently extend into colorectal and ovarian cancer.

### Statistical considerations and sample size

For stage 1 of the study, 5 samples are processed. This is sufficient to produce technical and biological replicates and undertake initial quality assessment.

Data handling and processing are performed as in stage 2.

For stage 2 of the study, initially 30 samples from benign and 30 from cancer patients are collected and processed. This data will inform the subsequent sample sizes for stages 3 and 4 of the overall study.

### Ethical approval

Ethical approval is sought from the National Research Ethics Service prior to the initiation of the study.

### Informed Consent process

Written informed consent is obtained from all patients participating in this study to collect their demographic and clinical data and for the blood sample and subsequent storage of the transcriptomic material and data. Where patients elect to do so, they are consented over the telephone. Patients can withdraw consent at any point. Anyone not deemed able to give informed consent are excluded from the study.

### Quality control

In order to assess that samples were labelled correctly and that there are no handling issues with each sample, an in silico quality control test is performed. This test is made up of checks for known genes that should be present in all samples as well as clustering analysis to assess outliers which could represent sample contamination.

Genes present in the sample data that should not be present are also looked for. For example, genes from other species.

### EXAMPLE 2

### Blood sample collection procedure for full-length RNA extraction

cDNA is DNA synthesized from a RNA template. Thus, the quality of the cDNA sample is related to the RNA from which it is reverse transcribed. Prior art processes for handling blood samples prior to RNA extraction involve overnight thawing of the frozen blood samples. The present inventors have found that this leads to significant RNA degradation which negatively impacts long-read sequencing. The present inventors use the following protocol to process blood samples prior to RNA extraction in order to minimize degradation and optimize RNA extraction for long-read sequencing.

Blood sample collection procedure for full-length RNA extraction
- Draw 2.5 ml of blood into the PAXgene Blood RNA Tube at room temperature (18-25°C). Gently invert the blood tube 10 times immediately after blood collection.
- If RNA is to be extracted on the same day as sample collection, store the blood sample upright at room temperature (18-25°C) for 2-3 hours, then continue with RNA extraction immediately using the PAXgene Blood RNA Kit.
   If RNA extraction is not carried out on the day of blood collection, follow instructions below for sample freezing, storage, and thawing:
- The blood sample should be stored at -20°C or below immediately after collection. For long-term storage, freeze the blood sample at -20°C for 24 hours before transferring to a -70°C or -80°C freezer.
- If the sample is to be transferred to a different location, ship on dry ice to make sure the sample stays frozen during transportation.
- On the day of RNA extraction, thaw the blood sample by placing the sample tube upright on a rack and incubating at room temperature (18-25°C) for 1-3 hours. Once the blood is fully thawed, gently invert the sample tube 10 times, incubate at room temperature for another 2 hours, then perform RNA extraction immediately using the PAXgene Blood RNA Kit.

As shown in Table 2 RNA integrity is improved with a shorter (3-hour) thawing period relative to overnight thawing. RNA Integrity Number was calculated as in Schroeder et al. (The RIN: an RNA integrity number for assigning integrity values to RNA measurements. BMC Molecular Biology 7, 3 (2006). https://doi.org/10.1186/1471-2199-7-3.) Samples were placed at -20°C within 12 hours for the "-20°C 2 weeks; overnight thawing" and "-20°C 1 month; 3-hour thawing" tests (second and third tests). 5 individuals were used for each test. All thawing was carried out at room temperature.

**Table 2 - blood sample storage conditions and RNA integrity**

| **Blood sample storage Condition** | **RNA Integrity Number** |
|---|---|
| Fresh | 8.3 ± 0.2 |
| -20°C 2 weeks; overnight thawing | 7.8 ± 0.2 |
| -20°C 1 month; 3-hour thawing | 8.4 ± 0.3 |
| 4°C 3 days, then -20°C 2 months; 3-hour thawing | 8.0 ± 0.3 |

## Claims

1. A method for discovering a disease biomarker comprising:
(i) providing a first cDNA sample from a subject with a disease and a second cDNA sample from a subject without the disease;
(ii) normalizing the first and the second cDNA samples;
(iii) sequencing the normalized first and second cDNA samples; and
(iv) comparing the sequencing output for the first and second cDNA samples to discover a disease biomarker; wherein:
the first and second cDNA samples comprise double stranded cDNA templates, each template having a known 5' pre-attached adapter and a known 3' pre-attached adapter;
and wherein normalizing the first and second cDNA samples comprises:
(i) denaturing the cDNA sample to produce single stranded cDNA templates;
(ii) re-associating the cDNA sample to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA sample using primers specific to the ligated oligonucleotides.

2. A method for diagnosing a disease in a subject comprising:
(i) providing a cDNA sample from the subject;
(ii) normalizing the cDNA sample; and
(iii) sequencing the normalized cDNA sample, wherein the sequencing output is used to identify whether the subject has the disease; wherein:
the cDNA sample comprises double stranded cDNA templates, each template having a known 5' pre-attached adapter and a known 3' pre-attached adapter;
and wherein normalizing the cDNA sample comprises:
(i) denaturing the cDNA sample to produce single stranded cDNA templates;
(ii) re-associating the cDNA sample to produce a mixture of post-association single stranded cDNA templates and post-association double stranded cDNA templates;
(iii) annealing a 5' adapter complex to the 5' pre-attached adapter of at least one post-association single stranded cDNA template, and annealing a 3' adapter complex to the 3' pre-attached adapter of the same post-association single stranded cDNA template, wherein each adapter complex comprises at least one oligonucleotide;
(iv) ligating an oligonucleotide from the 5' adapter complex to the 5' pre-attached adapter of the post-association single stranded cDNA template and ligating an oligonucleotide from the 3' adapter complex to the 3' pre-attached adapter of the same post-association single stranded cDNA template; and
(v) selectively amplifying the cDNA sample using primers specific to the ligated oligonucleotides;
and wherein the method further comprises comparing the sequencing output for the normalized cDNA sample to one or more reference sequences or to the sequencing output of one or more control samples.

3. The method of claim 2, wherein:
(a) the one or more control samples are from one or more subjects with and/or without the disease;
(b) the method further comprises reporting to the subject the outcome of the method; and/or
(c) the cDNA sample is obtained from a biological fluid or a fluid or lysate generated from a biological material, optionally wherein the cDNA sample is obtained from blood.

4. The method of any of claims 1 to 3, wherein:
(i) the disease is cancer; and/or
(ii) normalizing comprises increasing the amount of low abundance cDNA within each cDNA sample.

5. The method of any of claims 1 to 4 wherein:
(A) the 5' adapter complex is a front oligonucleotide dimer comprising:
(i) a front lig-oligonucleotide for ligating to the 5' pre-attached adapter of the (post-association) single stranded cDNA template; and
(ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
such that, on annealing, an end of the front lig-oligonucleotide is adjacent an end of the 5' pre-attached adapter to enable ligation of the front lig-oligonucleotide to the 5' pre-attached adapter at a ligation site; and
(B) the 3' adapter complex is a back oligonucleotide dimer comprising:
(i) a back lig-oligonucleotide for ligating to the 3' pre-attached adapter of the (post-association) single stranded cDNA template; and
(ii) a back link-oligonucleotide for annealing to the 3' pre-attached adapter and the back lig-oligonucleotide, the back link-oligonucleotide comprising a region complementary to the 3' pre-attached adapter and a region complementary to the back lig-oligonucleotide,
such that, on annealing, an end of the back lig-oligonucleotide is adjacent an end of the 3' pre-attached adapter to enable ligation of the back lig-oligonucleotide to the 3' pre-attached adapter at a ligation site,
optionally wherein:
(A) the front link-oligonucleotide comprises:
(i) a template overhang region at an end of the front link-oligonucleotide proximal the region complementary to the 5' pre-attached adapter, the template overhang region being non-complementary to a corresponding region of the (post-association) single stranded cDNA template; and/or
(ii) a lig-oligonucleotide overhang region at an end of the front link-oligonucleotide proximal the region complementary to the front lig-oligonucleotide, the lig-oligonucleotide overhang region being non-complementary to a corresponding region of the front lig-oligonucleotide; and/or
(B) the back link-oligonucleotide comprises:
(i) a template overhang region at an end of the back link-oligonucleotide proximal the region complementary to the 3' pre-attached adapter, the template overhang region being non-complementary to a corresponding region of the (post-association) single stranded cDNA template; and/or
(ii) a lig-oligonucleotide overhang region at an end of the back link-oligonucleotide proximal the region complementary to the back lig-oligonucleotide, the lig-oligonucleotide overhang region being non-complementary to a corresponding region of the back lig-oligonucleotide.

6. The method of any previous claim wherein sequencing comprises the use of long read sequencing.

7. The method of any of claims 1 or 4 to 6, wherein the first cDNA sample and the second cDNA sample are obtained from biological fluid or a fluid or lysate generated from a biological material, optionally wherein the first cDNA sample and the second cDNA sample are obtained from blood;
and/or wherein prior to step (i), the method comprises extracting RNA from biological fluid or a fluid or lysate generated from a biological material from the subject with a disease and from the subject without the disease and synthesizing cDNA using the RNA as a template.

8. The method of any of claims 2 to 6, further comprising, prior to step (i), extracting RNA from biological fluid or a fluid or lysate generated from a biological material from the subject and synthesizing cDNA using the RNA as a template.

9. The method of any of claims 1 or 4 to 7, wherein the disease biomarker is a cDNA sequence that is present in the first cDNA sample but not in the second cDNA sample or is present in the second cDNA sample but not in the first cDNA sample.

10. The method of any of claims 2 to 6 or 8 wherein the cDNA sample is obtained from blood by a method for processing a blood sample comprising:
(i) storing the blood sample at -15°C or below;
(ii) thawing the blood sample at 5 to 30°C for at least 1 hour;
(iii) extracting RNA from the thawed blood sample; and
(iv) synthesizing cDNA using the extracted RNA as a template.

11. The method of any of claims 1, 4 to 7 or 9 wherein the first cDNA sample and the second cDNA sample are obtained from blood by a method for processing a blood sample comprising:
(i) storing the blood sample at -15°C or below;
(ii) thawing the blood sample at 5 to 30°C for at least 1 hour;
(iii) extracting RNA from the thawed blood sample; and
(iv) synthesizing cDNA using the extracted RNA as a template.

12. The method of claim 10 or 11 wherein:
(a) the blood sample is:
(i) stored at -20°C or below;
(ii) thawed at 18 to 25°C; and/or
(iii) thawed for 1 to 4 hours;
and/or
(b) the blood sample is stored at -15°C or below or -20°C or below within 12 hours of collection.

13. Use in a method for discovering a cancer biomarker of an oligonucleotide dimer composition for selective amplification of single stranded cDNA by ligation of an oligonucleotide to a 5' and a 3' end of a post-association single stranded cDNA template having known 5' and 3' pre-attached adapters, wherein the composition comprises:
(A) a front oligonucleotide dimer comprising:
(i) a front lig-oligonucleotide for ligating to the 5' pre-attached adapter of the post-association single stranded cDNA template; and
(ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
(i) a back lig-oligonucleotide for ligating to the 3' pre-attached adapter of the post-association single stranded cDNA template; and
(ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
such that, on annealing, an end of the back lig-oligonucleotide is adjacent an end of the 3' pre-attached adapter to enable ligation of the back lig-oligonucleotide to the 3' pre-attached adapter at a ligation site,
optionally wherein:
(A) the front link-oligonucleotide comprises:
(i) a template overhang region at an end of the front link-oligonucleotide proximal the region complementary to the 5' pre-attached adapter, the template overhang region being non-complementary to a corresponding region of the post-association single stranded cDNA template; and/or
(ii) a lig-oligonucleotide overhang region at an end of the front link-oligonucleotide proximal the region complementary to the front lig-oligonucleotide, the lig-oligonucleotide overhang region being non-complementary to a corresponding region of the front lig-oligonucleotide; and/or
(B) the back link-oligonucleotide comprises:
(i) a template overhang region at an end of the back link-oligonucleotide proximal the region complementary to the 3' pre-attached adapter, the template overhang region being non-complementary to a corresponding region of the post-association single stranded cDNA template; and/or
(ii) a lig-oligonucleotide overhang region at an end of the back link-oligonucleotide proximal the region complementary to the back lig-oligonucleotide, the lig-oligonucleotide overhang region being non-complementary to a corresponding region of the back lig-oligonucleotide.

14. A system for discovering a disease biomarker, comprising:
(a) one or more testing devices for normalizing a first cDNA sample from a subject with a disease and a second cDNA sample from a subject without the disease and sequencing the normalized first and second cDNA samples;
(b) a processor; and
(c) storage medium comprising a computer application that, when executed by the processor, is configured to:
(i) access the determined sequence(s) for the first and second cDNA samples on the one or more testing devices
(ii) calculate whether there is a cDNA sequence that is present in the first cDNA sample but not in the second cDNA sample or is present in the second cDNA sample but not in the first cDNA sample; and
(iii) output from the processor the result of step (ii);
wherein the one or more testing devices comprises an oligonucleotide dimer composition for selective amplification of single stranded cDNA by ligation of an oligonucleotide to a 5' and a 3' end of a single stranded cDNA template having known 5' and 3' pre-attached adapters, wherein the oligonucleotide dimer composition comprises:
(A) a front oligonucleotide dimer comprising:
(i) a front lig-oligonucleotide for ligating to the 5' pre-attached adapter of the post-association single stranded cDNA template; and
(ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
(i) a back lig-oligonucleotide for ligating to the 3' pre-attached adapter of the post-association single stranded cDNA template; and
(ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
such that, on annealing, an end of the back lig-oligonucleotide is adjacent an end of the 3' pre-attached adapter to enable ligation of the back lig-oligonucleotide to the 3' pre-attached adapter at a ligation site.

15. A system for diagnosing a disease in a subject, comprising:
(a) one or more testing devices for normalizing a cDNA sample from a subject and sequencing the normalized cDNA sample
(b) a processor; and
(c) storage medium comprising a computer application that, when executed by the processor, is configured to:
(i) access the determined sequence(s) for the cDNA sample on the one or more testing devices
(ii) calculate whether a cDNA sequence is present or absent, wherein the presence or absence of the cDNA sequence is associated with the disease; and
(iii) output from the processor whether a subject has the disease;
wherein the one or more testing devices comprises an oligonucleotide dimer composition for selective amplification of single stranded cDNA by ligation of an oligonucleotide to a 5' and a 3' end of a single stranded cDNA template having known 5' and 3' pre-attached adapters,
wherein the oligonucleotide dimer composition comprises:
(A) a front oligonucleotide dimer comprising:
(i) a front lig-oligonucleotide for ligating to the 5' pre-attached adapter of the post-association single stranded cDNA template; and
(ii) a front link-oligonucleotide for annealing to the 5' pre-attached adapter and the front lig-oligonucleotide, the front link-oligonucleotide comprising a region complementary to the 5' pre-attached adapter and a region complementary to the front lig-oligonucleotide,
such that, on annealing, an end of the front lig-oligonucleotide is adjacent an end of the 5' pre-attached adapter to enable ligation of the front lig-oligonucleotide to the 5' pre-attached adapter at a ligation site; and
(B) a back oligonucleotide dimer comprising:
(i) a back lig-oligonucleotide for ligating to the 3' pre-attached adapter of the post-association single stranded cDNA template; and
(ii) a back link-oligonucleotide for annealing to the 3' pre-attached adapter and the back lig-oligonucleotide, the back link oligonucleotide comprising a region complementary to the 3' pre-attached adapter and a region complementary to the back lig-oligonucleotide,
such that, on annealing, an end of the back lig-oligonucleotide is adjacent an end of the 3' pre-attached adapter to enable ligation of the back lig-oligonucleotide to the 3' pre-attached adapter at a ligation site.

## Patentansprüche

1. Verfahren zum Identifizieren eines Krankheitsbiomarkers, umfassend:
(i) Bereitstellen einer ersten cDNA-Probe eines Subjekts mit einer Erkrankung und einer zweiten cDNA-Probe eines Subjekts ohne der Erkrankung;
(ii) Normalisieren der ersten und der zweiten cDNA-Probe;
(iii) Sequenzieren der normalisierten ersten und zweiten cDNA-Probe; und
(iv) Vergleichen des Sequenzierungsergebnisses der ersten und der zweiten cDNA-Probe, um einen Krankheitsbiomarker zu identifizieren; wobei:
die erste und die zweite cDNA-Probe doppelsträngige cDNA-Matrizen umfassen, wobei jede Matrize einen bekannten, vorab angefügten 5'-Adapter und einen bekannten, vorab angefügten 3'-Adapter aufweist;
und wobei das Normalisieren der ersten und der zweiten cDNA-Probe umfasst:
(i) Denaturieren der cDNA-Probe, um einzelsträngige cDNA-Matrizen zu erzeugen;
(ii) Reassoziieren der cDNA-Probe, um ein Gemisch aus nach der Assoziation vorliegenden einzelsträngigen cDNA-Matrizen und nach der Assoziation vorliegenden doppelsträngigen cDNA-Matrizen zu erzeugen;
(iii) Annealing eines 5'-Adapterkomplexes an den vorab angefügten 5'-Adapter mindestens einer nach der Assoziation vorliegenden einzelsträngigen cDNA-Matrize und Annealing eines 3'-Adapterkomplexes an den vorab angefügten 3'-Adapter derselben, nach der Assoziation vorliegenden einzelsträngigen cDNA-Matrize, wobei jeder Adapterkomplex mindestens ein Oligonukleotid umfasst;
(iv) Ligieren eines Oligonukleotids aus dem 5'-Adapterkomplex an den vorab angefügten 5'-Adapter der nach der Assoziation vorliegenden einzelsträngigen cDNA-Matrize und Ligieren eines Oligonukleotids aus dem 3'-Adapterkomplex an den vorab angefügten 3'-Adapter derselben, nach der Assoziation vorliegenden einzelsträngigen cDNA-Matrize; und
(v) selektives Amplifizieren der cDNA-Probe unter Verwendung von Primern, die für die ligierten Oligonukleotide spezifisch sind.

2. Verfahren zum Diagnostizieren einer Erkrankung bei einem Subjekt, umfassend:
(i) Bereitstellen einer cDNA-Probe des Subjekts;
(ii) Normalisieren der cDNA-Probe; und
(iii) Sequenzieren der normalisierten cDNA-Probe, wobei das Sequenzierungsergebnis verwendet wird, um zu ermitteln, ob die Erkrankung bei dem Subjekt vorliegt; wobei:
die cDNA-Probe doppelsträngige cDNA-Matrizen umfasst, wobei jede Matrize einen bekannten, vorab angefügten 5'-Adapter und einen bekannten, vorab angefügten 3'-Adapter aufweist;
und wobei das Normalisieren der cDNA-Probe umfasst:
(i) Denaturieren der cDNA-Probe, um einzelsträngige cDNA-Matrizen zu erzeugen;
(ii) Reassoziieren der cDNA-Probe, um ein Gemisch aus nach der Assoziation vorliegenden einzelsträngigen cDNA-Matrizen und nach der Assoziation vorliegenden doppelsträngigen cDNA-Matrizen zu erzeugen;
(iii) Annealing eines 5'-Adapterkomplexes an den vorab angefügten 5'-Adapter mindestens einer nach der Assoziation vorliegenden einzelsträngigen cDNA-Matrize und Annealing eines 3'-Adapterkomplexes an den vorab angefügten 3'-Adapter derselben, nach der Assoziation vorliegenden einzelsträngigen cDNA-Matrize, wobei jeder Adapterkomplex mindestens ein Oligonukleotid umfasst;
(iv) Ligieren eines Oligonukleotids aus dem 5'-Adapterkomplex an den vorab angefügten 5'-Adapter der nach der Assoziation vorliegenden einzelsträngigen cDNA-Matrize und Ligieren eines Oligonukleotids aus dem 3'-Adapterkomplex an den vorab angefügten 3'-Adapter derselben, nach der Assoziation vorliegenden einzelsträngigen cDNA-Matrize; und
(v) selektives Amplifizieren der cDNA-Probe unter Verwendung von Primern, die für die ligierten Oligonukleotide spezifisch sind;
und wobei das Verfahren weiter das Vergleichen des Sequenzierungsergebnisses für die normalisierte cDNA-Probe mit einer oder mehreren Referenzsequenzen oder mit dem Sequenzierungsergebnis einer oder mehrerer Kontrollproben umfasst.

3. Verfahren nach Anspruch 2, wobei:
(a) die eine oder die mehreren Kontrollproben von einem oder mehreren Subjekten mit und/oder ohne der Erkrankung stammen;
b) das Verfahren weiter das Mitteilen des Ergebnisses des Verfahrens an das Subjekt umfasst; und/oder
(c) die cDNA-Probe aus einem biologischen Fluid oder einem aus einem biologischen Material gewonnenen Fluid oder Lysat erhalten wird, wahlweise wobei die cDNA-Probe aus Blut erhalten wird.

4. Verfahren nach einem der Ansprüche 1 bis 3, wobei:
(i) die Erkrankung Krebs ist; und/oder
(ii) das Normalisieren das Erhöhen der Menge an cDNA von geringer Häufigkeit innerhalb jeder cDNA-Probe umfasst.

5. Verfahren nach einem der Ansprüche 1 bis 4, wobei:
(A) der 5'-Adapterkomplex ein vorderes Oligonukleotid-Dimer ist, welches umfasst:
(i) ein vorderes Lig-Oligonukleotid zum Ligieren an den vorab angefügten 5'-Adapter der (nach Assoziation vorliegenden) einzelsträngigen cDNA-Matrize; und
(ii) ein vorderes Link-Oligonukleotid zum Annealing an den vorab angefügten 5'-Adapter und das vordere Lig-Oligonukleotid, wobei das vordere Link-Oligonukleotid eine zu dem vorab angefügten 5'-Adapter komplementäre Region und eine zu dem vorderen Lig-Oligonukleotid komplementäre Region umfasst,
sodass beim Annealing ein Ende des vorderen Lig-Oligonukleotids an ein Ende des vorab angefügten 5'-Adapters angrenzt, um Ligation des vorderen Lig-Oligonukleotids an einer Ligationsstelle an den vorab angefügten 5'-Adapter zu ermöglichen; und
(B) der 3'-Adapterkomplex ein hinteres Oligonukleotid-Dimer ist, welches umfasst:
(i) ein hinteres Lig-Oligonukleotid zum Ligieren an den vorab angefügten 3'-Adapter der (nach Assoziation vorliegenden) einzelsträngigen cDNA-Matrize; und
(ii) ein hinteres Link-Oligonukleotid zum Annealing an den vorab angefügten 3'-Adapter und das hintere Lig-Oligonukleotid, wobei das hintere Link-Oligonukleotid eine zu dem vorab angefügten 3'-Adapter komplementäre Region und eine zu dem hinteren Lig-Oligonukleotid komplementäre Region umfasst,
sodass beim Annealing ein Ende des hinteren Lig-Oligonukleotids an ein Ende des vorab angefügten 3'-Adapters angrenzt, um Ligation des hinteren Lig-Oligonukleotids an einer Ligationsstelle an den vorab angefügten 3'-Adapter zu ermöglichen,
wahlweise wobei:
(A) das vordere Link-Oligonukleotid umfasst:
(i) eine Matrizen-Überhangregion an einem Ende des vorderen Link-Oligonukleotids proximal der Region, die zu dem vorab angefügten 5'-Adapter komplementär ist, wobei die Matrizen-Überhangregion zu einer entsprechenden Region der (nach Assoziation vorliegenden) einzelsträngigen cDNA-Matrize nicht komplementär ist; und/oder
(ii) eine Lig-Oligonukleotid-Überhangregion an einem Ende des vorderen Link-Oligonukleotids proximal der Region, die zu dem vorderen Lig-Oligonukleotid komplementär ist, wobei die Lig-Oligonukleotid-Überhangregion zu einer entsprechenden Region des vorderen Lig-Oligonukleotids nicht komplementär ist; und/oder
(B) das hintere Link-Oligonukleotid umfasst:
(i) eine Matrizen-Überhangregion an einem Ende des hinteren Link-Oligonukleotids proximal der Region, die zu dem vorab angefügten 3'-Adapter komplementär ist, wobei die Matrizen-Überhangregion zu einer entsprechenden Region der (nach Assoziation vorliegenden) einzelsträngigen cDNA-Matrize nicht komplementär ist; und/oder
(ii) eine Lig-Oligonukleotid-Überhangregion an einem Ende des hinteren Link-Oligonukleotids proximal der Region, die zu dem hinteren Lig-Oligonukleotid komplementär ist, wobei die Lig-Oligonukleotid-Überhangregion zu einer entsprechenden Region des hinteren Lig-Oligonukleotids nicht komplementär ist.

6. Verfahren nach einem vorstehenden Anspruch, wobei das Sequenzieren die Verwendung von Long-Read-Sequenzierung umfasst.

7. Verfahren nach einem der Ansprüche 1 oder 4 bis 6, wobei die erste cDNA-Probe und die zweite cDNA-Probe aus biologischem Fluid oder einem aus einem biologischem Material gewonnenen Fluid oder Lysat erhalten werden, wahlweise wobei die erste cDNA-Probe und die zweite cDNA-Probe aus Blut erhalten werden;
und/oder wobei das Verfahren vor Schritt (i) das Extrahieren von RNA aus biologischem Fluid oder einem aus einem biologischem Material gewonnenen Fluid oder Lysat des Subjekts mit einer Erkrankung und des Subjekts ohne der Erkrankung und das Synthetisieren von cDNA unter Verwendung der RNA als Matrize umfasst.

8. Verfahren nach einem der Ansprüche 2 bis 6, das vor Schritt (i) weiter das Extrahieren von RNA aus biologischen Fluid oder einem aus einem biologischen Material gewonnenen Fluid oder Lysat des Subjekts und das Synthetisieren von cDNA unter Verwendung der RNA als Matrize umfasst.

9. Verfahren nach einem der Ansprüche 1 oder 4 bis 7, wobei der Krankheitsbiomarker eine cDNA-Sequenz ist, die in der ersten cDNA-Probe, aber nicht in der zweiten cDNA-Probe vorhanden ist oder in der zweiten cDNA-Probe, aber nicht in der ersten cDNA-Probe vorhanden ist.

10. Verfahren nach einem der Ansprüche 2 bis 6 oder 8, wobei die cDNA-Probe aus Blut erhalten wird durch ein Verfahren zum Verarbeiten einer Blutprobe, welches umfasst:
(i) Lagern der Blutprobe bei -15 °C oder darunter;
(ii) Auftauen der Blutprobe bei 5 bis 30 °C über mindestens 1 Stunde;
(iii) Extrahieren von RNA aus der aufgetauten Blutprobe; und
(iv) Synthetisieren von cDNA unter Verwendung der extrahierten RNA als Matrize.

11. Verfahren nach einem der Ansprüche 1, 4 bis 7 oder 9, wobei die erste cDNA-Probe und die zweite cDNA-Probe aus Blut erhalten werden durch ein Verfahren zum Verarbeiten einer Blutprobe, welches umfasst:
(i) Lagern der Blutprobe bei -15 °C oder darunter;
(ii) Auftauen der Blutprobe bei 5 bis 30 °C über mindestens 1 Stunde;
(iii) Extrahieren von RNA aus der aufgetauten Blutprobe; und
(iv) Synthetisieren von cDNA unter Verwendung der extrahierten RNA als Matrize.

12. Verfahren nach Anspruch 10 oder 11, wobei:
(a) die Blutprobe:
(i) bei -20 °C oder darunter gelagert wird;
(ii) bei 18 bis 25 °C aufgetaut wird; und/oder
(iii) über 1 bis 4 Stunden aufgetaut wird;
und/oder
b) die Blutprobe innerhalb von 12 Stunden nach Entnahme bei -15 °C oder darunter oder - 20 °C oder darunter gelagert wird.

13. Verwendung, in einem Verfahren zum Identifizieren eines Krebsbiomarkers, einer Oligonukleotid-Dimer-Zusammensetzung zur selektiven Amplifikation von einzelsträngiger cDNA durch Ligation eines Oligonukleotids an ein 5'- und ein 3'-Ende einer nach Assoziation vorliegenden einzelsträngigen cDNA-Matrize, die bekannte, vorab angefügte 5'- und 3'-Adapter aufweist, wobei die Zusammensetzung umfasst:
(A) ein vorderes Oligonukleotid-Dimer, welches umfasst:
(i) ein vorderes Lig-Oligonukleotid zum Ligieren an den vorab angefügten 5'-Adapter der nach Assoziation vorliegenden einzelsträngigen cDNA-Matrize; und
(ii) ein vorderes Link-Oligonukleotid zum Annealing an den vorab angefügten 5'-Adapter und das vordere Lig-Oligonukleotid, wobei das vordere Link-Oligonukleotid eine zu dem vorab angefügten 5'-Adapter komplementäre Region und eine zu dem vorderen Lig-Oligonukleotid komplementäre Region umfasst,
sodass beim Annealing ein Ende des vorderen Lig-Oligonukleotids an ein Ende des vorab angefügten 5'-Adapters angrenzt, um Ligation des vorderen Lig-Oligonukleotids an einer Ligationsstelle an den vorab angefügten 5'-Adapter zu ermöglichen; und
(B) ein hinteres Oligonukleotid-Dimer, welches umfasst:
(i) ein hinteres Lig-Oligonukleotid zum Ligieren an den vorab angefügten 3'-Adapter der nach Assoziation vorliegenden einzelsträngigen cDNA-Matrize; und
(ii) ein hinteres Link-Oligonukleotid zum Annealing an den vorab angefügten 3'-Adapter und das hintere Lig-Oligonukleotid, wobei das hintere Link-Oligonukleotid eine zu dem vorab angefügten 3'-Adapter komplementäre Region und eine zu dem hinteren Lig-Oligonukleotid komplementäre Region umfasst,
sodass beim Annealing ein Ende des hinteren Lig-Oligonukleotids an ein Ende des vorab angefügten 3'-Adapters angrenzt, um Ligation des hinteren Lig-Oligonukleotids an einer Ligationsstelle an den vorab angefügten 3'-Adapter zu ermöglichen,
wahlweise wobei:
(A) das vordere Link-Oligonukleotid umfasst:
(i) eine Matrizen-Überhangregion an einem Ende des vorderen Link-Oligonukleotids proximal der Region, die zu dem vorab angefügten 5'-Adapter komplementär ist, wobei die Matrizen-Überhangregion zu einer entsprechenden Region der nach Assoziation vorliegenden einzelsträngigen cDNA-Matrize nicht komplementär ist; und/oder
(ii) eine Lig-Oligonukleotid-Überhangregion an einem Ende des vorderen Link-Oligonukleotids proximal der Region, die zu dem vorderen Lig-Oligonukleotid komplementär ist, wobei die Lig-Oligonukleotid-Überhangregion zu einer entsprechenden Region des vorderen Lig-Oligonukleotids nicht komplementär ist; und/oder
(B) das hintere Link-Oligonukleotid umfasst:
(i) eine Matrizen-Überhangregion an einem Ende des hinteren Link-Oligonukleotids proximal der Region, die zu dem vorab angefügten 3'-Adapter komplementär ist, wobei die Matrizen-Überhangregion zu einer entsprechenden Region der nach Assoziation vorliegenden einzelsträngigen cDNA-Matrize nicht komplementär ist; und/oder
(ii) eine Lig-Oligonukleotid-Überhangregion an einem Ende des hinteren Lig-Oligonukleotids proximal der Region, die zu dem hinteren Lig-Oligonukleotid komplementär ist, wobei die Lig-Oligonukleotid-Überhangregion zu einer entsprechenden Region des hinteren Lig-Oligonukleotids nicht komplementär ist.

14. System zum Identifizieren eines Krankheitsbiomarkers, umfassend:
(a) eine oder mehrere Testvorrichtungen zum Normalisieren einer ersten cDNA-Probe eines Subjekts mit einer Erkrankung und einer zweiten cDNA-Probe eines Subjekts ohne der Erkrankung und zum Sequenzieren der normalisierten ersten und zweiten cDNA-Probe;
(b) einen Prozessor; und
(c) ein Speichermedium, das eine Computeranwendung umfasst, die so konfiguriert ist, dass sie, wenn sie von dem Prozessor ausgeführt wird:
(i) auf die bestimmte(n) Sequenz(en) der ersten und der zweiten cDNA-Probe auf der einen oder den mehreren Testvorrichtungen zugreift,
(ii) berechnet, ob es eine cDNA-Sequenz gibt, die in der ersten cDNA-Probe, aber nicht in der zweiten cDNA-Probe vorhanden ist oder in der zweiten cDNA-Probe, aber nicht in der ersten cDNA-Probe vorhanden ist; und
(iii) das Ergebnis aus Schritt (ii) aus dem Prozessor ausgibt;
wobei die eine oder die mehreren Testvorrichtungen eine Oligonukleotid-Dimer-Zusammensetzung zur selektiven Amplifikation von einzelsträngiger cDNA durch Ligation eines Oligonukleotids an ein 5'- und ein 3'-Ende einer einzelsträngigen cDNA-Matrize, die bekannte, vorab angefügte 5'- und 3'-Adapter aufweist, umfasst, wobei die Oligonukleotid-Dimer-Zusammensetzung umfasst:
(A) ein vorderes Oligonukleotid-Dimer, welches umfasst:
(i) ein vorderes Lig-Oligonukleotid zum Ligieren an den vorab angefügten 5'-Adapter der nach Assoziation vorliegenden einzelsträngigen cDNA-Matrize; und
(ii) ein vorderes Link-Oligonukleotid zum Annealing an den vorab angefügten 5'-Adapter und das vordere Lig-Oligonukleotid, wobei das vordere Link-Oligonukleotid eine
zu dem vorab angefügten 5'-Adapter komplementäre Region und eine zu dem vorderen Lig-Oligonukleotid komplementäre Region umfasst,
sodass beim Annealing ein Ende des vorderen Lig-Oligonukleotids an ein Ende des vorab angefügten 5'-Adapters angrenzt, um Ligation des vorderen Lig-Oligonukleotids an einer Ligationsstelle an den vorab angefügten 5'-Adapter zu ermöglichen; und
(B) ein hinteres Oligonukleotid-Dimer, welches umfasst:
(i) ein hinteres Lig-Oligonukleotid zum Ligieren an den vorab angefügten 3'-Adapter der nach Assoziation vorliegenden einzelsträngigen cDNA-Matrize; und
(ii) ein hinteres Link-Oligonukleotid zum Annealing an den vorab angefügten 3'-Adapter und das hintere Lig-Oligonukleotid, wobei das hintere Link-Oligonukleotid eine zu dem vorab angefügten 3'-Adapter komplementäre Region und eine zu dem hinteren Lig-Oligonukleotid komplementäre Region umfasst,
sodass beim Annealing ein Ende des hinteren Lig-Oligonukleotids an ein Ende des vorab angefügten 3'-Adapters angrenzt, um Ligation des hinteren Lig-Oligonukleotids an einer Ligationsstelle an den vorab angefügten 3'-Adapter zu ermöglichen.

15. System zum Diagnostizieren einer Erkrankung bei einem Subjekt, umfassend:
(a) eine oder mehrere Testvorrichtungen zum Normalisieren einer cDNA-Probe eines Subjekts und zum Sequenzieren der normalisierten cDNA-Probe;
(b) einen Prozessor; und
(c) ein Speichermedium, das eine Computeranwendung umfasst, die so konfiguriert ist, dass sie, wenn sie von dem Prozessor ausgeführt wird:
(i) auf die bestimmte(n) Sequenz(en) der cDNA-Probe auf der einen oder den mehreren Testvorrichtungen zugreift;
(ii) berechnet, ob eine cDNA-Sequenz vorhanden ist oder fehlt, wobei das Vorhandensein oder Fehlen der cDNA-Sequenz mit der Erkrankung assoziiert ist; und
(iii) aus dem Prozessor ausgibt, ob ein Subjekt die Erkrankung hat;
wobei die eine oder die mehreren Testvorrichtungen eine Oligonukleotid-Dimer-Zusammensetzung zur selektiven Amplifikation von einzelsträngiger cDNA durch Ligation eines Oligonukleotids an ein 5'- und ein 3'-Ende einer einzelsträngigen cDNA-Matrize, die bekannte, vorab angefügte 5'- und 3'-Adapter aufweist, umfasst, wobei die Oligonukleotid-Dimer-Zusammensetzung umfasst:
(A) ein vorderes Oligonukleotid-Dimer, welches umfasst:
(i) ein vorderes Lig-Oligonukleotid zum Ligieren an den vorab angefügten 5'-Adapter der nach Assoziation vorliegenden einzelsträngigen cDNA-Matrize; und
(ii) ein vorderes Link-Oligonukleotid zum Annealing an den vorab angefügten 5'-Adapter und das vordere Lig-Oligonukleotid, wobei das vordere Link-Oligonukleotid eine
zu dem vorab angefügten 5'-Adapter komplementäre Region und eine zu dem vorderen Lig-Oligonukleotid komplementäre Region umfasst,
sodass beim Annealing ein Ende des vorderen Lig-Oligonukleotids an ein Ende des vorab angefügten 5'-Adapters angrenzt, um Ligation des vorderen Lig-Oligonukleotids an einer Ligationsstelle an den vorab angefügten 5'-Adapter zu ermöglichen; und
(B) ein hinteres Oligonukleotid-Dimer, welches umfasst:
(i) ein hinteres Lig-Oligonukleotid zum Ligieren an den vorab angefügten 3'-Adapter der nach Assoziation vorliegenden einzelsträngigen cDNA-Matrize; und
(ii) ein hinteres Link-Oligonukleotid zum Annealing an den vorab angefügten 3'-Adapter und das hintere Lig-Oligonukleotid, wobei das hintere Link-Oligonukleotid eine zu dem vorab angefügten 3'-Adapter komplementäre Region und eine zu dem hinteren Lig-Oligonukleotid komplementäre Region umfasst,
sodass beim Annealing ein Ende des hinteren Lig-Oligonukleotids an ein Ende des vorab angefügten 3'-Adapters angrenzt, um Ligation des hinteren Lig-Oligonukleotids an einer Ligationsstelle an den vorab angefügten 3'-Adapter zu ermöglichen.

## Revendications

1. Procédé de découverte d'un biomarqueur de maladie comprenant :
(i) la fourniture d'un premier échantillon d'ADNc provenant d'un sujet atteint d'une maladie et d'un second échantillon d'ADNc provenant d'un sujet non atteint de la maladie ;
(ii) la normalisation du premier et du second échantillon d'ADNc ;
(iii) le séquençage des premier et second échantillons d'ADNc normalisés ; et
(iv) la comparaison de la sortie de séquençage des premier et second échantillons d'ADNc pour découvrir un biomarqueur de maladie ; dans lequel :
les premier et second échantillons d'ADNc comprennent des matrices d'ADNc double brin, chaque matrice présentant un adaptateur pré-attaché en 5' connu et un adaptateur pré-attaché en 3' connu ;
et dans lequel la normalisation des premier et second échantillons d'ADNc comprend :
(i) la dénaturation de l'échantillon d'ADNc pour produire des matrices d'ADNc simple brin ;
(ii) la réassociation de l'échantillon d'ADNc pour produire un mélange de matrices d'ADNc simple brin post-association et de matrices d'ADNc double brin post-association ;
(iii) l'hybridation d'un complexe adaptateur 5' à l'adaptateur pré-attaché en 5' d'au moins une matrice d'ADNc simple brin post-association, et l'hybridation d'un complexe adaptateur 3' à l'adaptateur pré-attaché en 3' de la même matrice d'ADNc simple brin post-association, dans lequel chaque complexe adaptateur comprend au moins un oligonucléotide ;
(iv) la ligation d'un oligonucléotide du complexe adaptateur 5' à l'adaptateur 5' pré-attaché de la matrice d'ADNc simple brin post-association et la ligation d'un oligonucléotide du complexe adaptateur 3' à l'adaptateur 3' pré-attaché de la même matrice d'ADNc simple brin post-association ; et
(v) l'amplification sélective de l'échantillon d'ADNc à l'aide d'amorces spécifiques aux oligonucléotides ligaturés.

2. Procédé de diagnostic d'une maladie chez un sujet comprenant :
(i) la fourniture d'un échantillon d'ADNc provenant du sujet ;
(ii) la normalisation de l'échantillon d'ADNc ; et
(iii) le séquençage de l'échantillon d'ADNc normalisé, dans lequel la sortie de séquençage est utilisée pour identifier si le sujet est atteint de la maladie ; dans lequel :
l'échantillon d'ADNc comprend des matrices d'ADNc double brin, chaque matrice présentant un adaptateur 5' pré-attaché connu et un adaptateur 3' pré-attaché connu ;
et dans lequel la normalisation de l'échantillon d'ADNc comprend :
(i) la dénaturation de l'échantillon d'ADNc pour produire des matrices d'ADNc simple brin ;
(ii) la réassociation de l'échantillon d'ADNc pour produire un mélange de matrices d'ADNc simple brin post-association et de matrices d'ADNc double brin post-association ;
(iii) l'hybridation d'un complexe adaptateur 5' à l'adaptateur pré-attaché en 5' d'au moins une matrice d'ADNc simple brin post-association, et l'hybridation d'un complexe adaptateur 3' à l'adaptateur pré-attaché en 3' de la même matrice d'ADNc simple brin post-association, dans lequel chaque complexe adaptateur comprend au moins un oligonucléotide ;
(iv) la ligation d'un oligonucléotide du complexe adaptateur 5' à l'adaptateur 5' pré-attaché de la matrice d'ADNc simple brin post-association et la ligation d'un oligonucléotide du complexe adaptateur 3' à l'adaptateur 3' pré-attaché de la même matrice d'ADNc simple brin post-association ; et
(v) l'amplification sélective de l'échantillon d'ADNc à l'aide d'amorces spécifiques aux oligonucléotides ligaturés ;
et dans lequel le procédé comprend en outre la comparaison de la sortie de séquençage de l'échantillon d'ADNc normalisé à une ou plusieurs séquences de référence ou à la sortie de séquençage d'un ou de plusieurs échantillons témoins.

3. Procédé selon la revendication 2, dans lequel :
(a) les un ou plusieurs échantillons témoins proviennent d'un ou de plusieurs sujets atteint et/ou non atteint de la maladie ;
(b) le procédé comprend en outre le compte-rendu au sujet du résultat du procédé ; et/ou
(c) l'échantillon d'ADNc est obtenu à partir d'un fluide biologique ou d'un fluide ou lysat généré à partir d'un matériel biologique, facultativement dans lequel l'échantillon d'ADNc est obtenu à partir de sang.

4. Procédé selon l'une quelconque des revendications 1 à 3, dans lequel :
(i) la maladie est le cancer ; et/ou
(ii) la normalisation comprend l'augmentation de la quantité d'ADNc à faible abondance dans chaque échantillon d'ADNc.

5. Procédé selon l'une quelconque des revendications 1 à 4, dans lequel :
(A) le complexe adaptateur 5' est un dimère d'oligonucléotide avant comprenant :
(i) un oligonucléotide de ligation avant pour une ligation à l'adaptateur pré-attaché en 5' de la matrice d'ADNc simple brin (post-association) ; et
(ii) un oligonucléotide de liaison avant pour s'hybrider à l'adaptateur pré-attaché en 5' et à l'oligonucléotide de ligation avant, l'oligonucléotide de liaison avant comprenant une région complémentaire à l'adaptateur pré-attaché en 5' et une région complémentaire à l'oligonucléotide de ligation avant,
de telle sorte que, lors de l'hybridation, une extrémité de l'oligonucléotide de ligation avant soit adjacente à une extrémité de l'adaptateur pré-attaché en 5' pour permettre une ligation de l'oligonucléotide de ligation avant à l'adaptateur pré-attaché en 5' au niveau d'un site de ligation ; et
(B) le complexe adaptateur 3' est un dimère d'oligonucléotide arrière comprenant :
(i) un oligonucléotide de ligation arrière pour une ligation à l'adaptateur pré-attaché en 3' de la matrice d'ADNc simple brin (post-association) ; et
(ii) un oligonucléotide de liaison arrière destiné à s'hybrider à l'adaptateur pré-attaché en 3' et à l'oligonucléotide de ligation arrière, l'oligonucléotide de liaison arrière comprenant une région complémentaire à l'adaptateur pré-attaché en 3' et une région complémentaire à l'oligonucléotide de ligation arrière,
de telle sorte que, lors de l'hybridation, une extrémité de l'oligonucléotide de ligation arrière soit adjacente à une extrémité de l'adaptateur pré-attaché en 3' pour permettre une ligation de l'oligonucléotide de ligation arrière à l'adaptateur pré-attaché en 3' au niveau d'un site de ligation,
facultativement dans lequel :
(A) l'oligonucléotide de liaison avant comprend :
(i) une région de surplomb de matrice à une extrémité de l'oligonucléotide de liaison avant proximale à la région complémentaire à l'adaptateur pré-attaché en 5', la région de surplomb de matrice n'étant pas complémentaire à une région correspondante de la matrice d'ADNc simple brin (post-association) ; et/ou
(ii) une région de surplomb d'oligonucléotide de ligation à une extrémité de l'oligonucléotide de liaison avant proximale à la région complémentaire à l'oligonucléotide de ligation avant, la région de surplomb d'oligonucléotide de ligation n'étant pas complémentaire à une région correspondante de l'oligonucléotide de ligation avant ; et/ou
(B) l'oligonucléotide de liaison arrière comprend :
(i) une région de surplomb de matrice à une extrémité de l'oligonucléotide de liaison arrière proximale à la région complémentaire à l'adaptateur pré-attaché en 3', la région de surplomb de matrice n'étant pas complémentaire à une région correspondante de la matrice d'ADNc simple brin (post-association) ; et/ou
(ii) une région de surplomb d'oligonucléotide de ligation à une extrémité de l'oligonucléotide de liaison arrière proximale à la région complémentaire à l'oligonucléotide de ligation arrière, la région de surplomb d'oligonucléotide de ligation n'étant pas complémentaire à une région correspondante de l'oligonucléotide de ligation arrière.

6. Procédé selon une quelconque revendication précédente, dans lequel le séquençage comprend l'utilisation du séquençage à lecture longue.

7. Procédé selon l'une quelconque des revendications 1 ou 4 à 6, dans lequel le premier échantillon d'ADNc et le second échantillon d'ADNc sont obtenus à partir d'un fluide biologique ou d'un fluide ou lysat généré à partir d'un matériel biologique, facultativement dans lequel le premier échantillon d'ADNc et le second échantillon d'ADNc sont obtenus à partir de sang ;
et/ou dans lequel, avant l'étape (i), le procédé comprend l'extraction d'ARN à partir d'un fluide biologique ou d'un fluide ou lysat généré à partir d'un matériel biologique provenant du sujet atteint d'une maladie et du sujet non atteint de la maladie et la synthèse d'ADNc en utilisant l'ARN comme matrice.

8. Procédé selon l'une quelconque des revendications 2 à 6, comprenant en outre, avant l'étape (i), l'extraction d'ARN à partir d'un fluide biologique ou d'un fluide ou lysat généré à partir d'un matériel biologique provenant du sujet et la synthèse d'ADNc en utilisant l'ARN comme matrice.

9. Procédé selon l'une quelconque des revendications 1 ou 4 à 7, dans lequel le biomarqueur de la maladie est une séquence d'ADNc qui est présente dans le premier échantillon d'ADNc mais pas dans le second échantillon d'ADNc ou est présente dans le second échantillon d'ADNc mais pas dans le premier échantillon d'ADNc.

10. Procédé selon l'une quelconque des revendications 2 à 6 ou 8, dans lequel l'échantillon d'ADNc est obtenu à partir du sang par un procédé de traitement d'un échantillon de sang comprenant :
(i) le stockage de l'échantillon de sang à -15 °C ou moins ;
(ii) la décongélation de l'échantillon de sang à 5 à 30 °C pendant au moins 1 heure ;
(iii) l'extraction de l'ARN de l'échantillon de sang décongelé ; et
(iv) la synthèse de l'ADNc en utilisant l'ARN extrait comme matrice.

11. Procédé selon l'une quelconque des revendications 1, 4 à 7 ou 9, dans lequel le premier échantillon d'ADNc et le second échantillon d'ADNc sont obtenus à partir du sang par un procédé de traitement d'un échantillon de sang comprenant :
(i) le stockage de l'échantillon de sang à -15 °C ou moins ;
(ii) la décongélation de l'échantillon de sang à 5 à 30 °C pendant au moins 1 heure ;
(iii) l'extraction de l'ARN de l'échantillon de sang décongelé ; et
(iv) la synthèse de l'ADNc en utilisant l'ARN extrait comme matrice.

12. Procédé selon la revendication 10 ou 11, dans lequel :
(a) l'échantillon de sang est :
(i) stocké à -20 °C ou moins ;
(ii) décongelé à 18 à 25 °C ; et/ou
(iii) décongelé pendant 1 à 4 heures ;
et/ou
(b) l'échantillon de sang est stocké à -15°C ou moins ou à -20°C ou moins dans les 12 heures suivant le prélèvement.

13. Utilisation, dans un procédé de découverte d'un biomarqueur du cancer, d'une composition de dimères d'oligonucléotide pour une amplification sélective d'ADNc simple brin par une ligation d'un oligonucléotide à une extrémité 5' et une extrémité 3' d'une matrice d'ADNc simple brin post-association comportant des adaptateurs pré-attachés en 5' et 3' connus, dans laquelle la composition comprend :
(A) un dimère d'oligonucléotide avant comprenant :
(i) un oligonucléotide de ligation avant pour une ligation à l'adaptateur pré-attaché en 5' de la matrice d'ADNc simple brin post-association ; et
(ii) un oligonucléotide de liaison avant pour s'hybrider à l'adaptateur pré-attaché en 5' et à l'oligonucléotide de ligation avant, l'oligonucléotide de liaison avant comprenant une région complémentaire à l'adaptateur pré-attaché en 5' et une région complémentaire à l'oligonucléotide de ligation avant,
de telle sorte que, lors de l'hybridation, une extrémité de l'oligonucléotide de ligation avant soit adjacente à une extrémité de l'adaptateur pré-attaché en 5' pour permettre une ligation de l'oligonucléotide de ligation avant à l'adaptateur pré-attaché en 5' au niveau d'un site de ligation ; et
(B) un dimère d'oligonucléotide arrière comprenant :
(i) un oligonucléotide de ligation arrière pour une ligation à l'adaptateur pré-attaché en 3' de la matrice d'ADNc simple brin post-association ; et
(ii) un oligonucléotide de liaison arrière pour s'hybrider à l'adaptateur pré-attaché en 3' et à l'oligonucléotide de ligation arrière, l'oligonucléotide de liaison arrière comprenant une région complémentaire à l'adaptateur pré-attaché en 3' et une région complémentaire à l'oligonucléotide de ligation arrière,
de telle sorte que, lors de l'hybridation, une extrémité de l'oligonucléotide de ligation arrière soit adjacente à une extrémité de l'adaptateur pré-attaché en 3' pour permettre une ligation de l'oligonucléotide de ligation arrière à l'adaptateur pré-attaché en 3' au niveau d'un site de ligation,
facultativement dans laquelle :
(A) l'oligonucléotide de liaison avant comprend :
(i) une région de surplomb de matrice à une extrémité de l'oligonucléotide de liaison avant proximale à la région complémentaire à l'adaptateur pré-attaché en 5', la région de surplomb de matrice n'étant pas complémentaire à une région correspondante de la matrice d'ADNc simple brin post-association ; et/ou
(ii) une région de surplomb d'oligonucléotide de ligation à une extrémité de l'oligonucléotide de liaison avant proximale à la région complémentaire à l'oligonucléotide de ligation avant, la région de surplomb d'oligonucléotide de ligation n'étant pas complémentaire à une région correspondante de l'oligonucléotide de ligation avant ; et/ou
(B) l'oligonucléotide de liaison arrière comprend :
(i) une région de surplomb de matrice à une extrémité de l'oligonucléotide de liaison arrière proximale à la région complémentaire à l'adaptateur pré-attaché en 3', la région de surplomb de matrice n'étant pas complémentaire à une région correspondante de la matrice d'ADNc simple brin post-association ; et/ou
(ii) une région de surplomb d'oligonucléotide de ligation à une extrémité de l'oligonucléotide de liaison arrière proximale à la région complémentaire à l'oligonucléotide de ligation arrière, la région de surplomb d'oligonucléotide de ligation n'étant pas complémentaire à une région correspondante de l'oligonucléotide de ligation arrière.

14. Système de découverte d'un biomarqueur de maladie, comprenant :
(a) un ou plusieurs dispositifs de test pour normaliser un premier échantillon d'ADNc provenant d'un sujet atteint d'une maladie et un second échantillon d'ADNc provenant d'un sujet non atteint de la maladie et séquencer les premier et second échantillons d'ADNc normalisés ;
b) un processeur ; et
(c) un support de stockage comprenant une application informatique qui, lorsqu'elle est exécutée par le processeur, est configurée pour :
(i) avoir accès à la ou aux séquences déterminées pour les premier et second échantillons d'ADNc sur les un ou plusieurs dispositifs de test
(ii) calculer s'il existe une séquence d'ADNc qui est présente dans le premier échantillon d'ADNc mais pas dans le second échantillon d'ADNc, ou est présente dans le second échantillon d'ADNc mais pas dans le premier échantillon d'ADNc ; et
(iii) sortir du processeur le résultat de l'étape (ii);
dans lequel les un ou plusieurs dispositifs de test comprennent une composition de dimère d'oligonucléotide pour une amplification sélective d'ADNc simple brin par une ligation d'un oligonucléotide à une extrémité 5' et une extrémité 3' d'une matrice d'ADNc simple brin comportant des adaptateurs pré-attachés en 5' et 3' connus, dans lequel la composition de dimère d'oligonucléotide comprend :
(A) un dimère d'oligonucléotide avant comprenant :
(i) un oligonucléotide de ligation avant pour une ligation à l'adaptateur pré-attaché en 5' de la matrice d'ADNc simple brin post-association ; et
(ii) un oligonucléotide de liaison avant pour s'hybrider à l'adaptateur pré-attaché en 5' et à l'oligonucléotide de ligation avant, l'oligonucléotide de liaison avant comprenant une région
complémentaire à l'adaptateur pré-attaché en 5' et une région complémentaire à l'oligonucléotide de ligation avant,
de telle sorte que, lors de l'hybridation, une extrémité de l'oligonucléotide de ligation avant soit adjacente à une extrémité de l'adaptateur pré-attaché en 5' pour permettre une ligation de l'oligonucléotide de ligation avant à l'adaptateur pré-attaché en 5' au niveau d'un site de ligation ; et
(B) un dimère d'oligonucléotide arrière comprenant :
(i) un oligonucléotide de ligation arrière pour une ligation à l'adaptateur pré-attaché en 3' de la matrice d'ADNc simple brin post-association ; et
(ii) un oligonucléotide de liaison arrière pour s'hybrider à l'adaptateur pré-attaché en 3' et à l'oligonucléotide de ligation arrière, l'oligonucléotide de liaison arrière comprenant une région complémentaire à l'adaptateur pré-attaché en 3' et une région complémentaire à l'oligonucléotide de ligation arrière,
de telle sorte que, lors de l'hybridation, une extrémité de l'oligonucléotide de ligation arrière soit adjacente à une extrémité de l'adaptateur pré-attaché en 3' pour permettre une ligation de l'oligonucléotide de ligation arrière à l'adaptateur pré-attaché en 3' au niveau d'un site de ligation.

15. Système de diagnostic d'une maladie chez un sujet, comprenant :
(a) un ou plusieurs dispositifs de test pour normaliser un échantillon d'ADNc provenant d'un sujet et séquencer l'échantillon d'ADNc normalisé
(b) un processeur ; et
(c) un support de stockage comprenant une application informatique qui, lorsqu'elle est exécutée par le processeur, est configurée pour :
(i) avoir accès à la ou aux séquences déterminées pour l'échantillon d'ADNc sur les un ou plusieurs dispositifs de test
(ii) calculer si une séquence d'ADNc est présente ou absente, dans lequel la présence ou l'absence de la séquence d'ADNc est associée à la maladie ; et
(iii) sortir du processeur si un sujet est atteint de la maladie ;
dans lequel les un ou plusieurs dispositifs de test comprennent une composition de dimère d'oligonucléotide pour une amplification sélective d'ADNc simple brin par une ligation d'un oligonucléotide à une extrémité 5' et une extrémité 3' d'une matrice d'ADNc simple brin comportant des adaptateurs pré-attachés en 5' et 3' connus, dans lequel la composition de dimère d'oligonucléotide comprend :
(A) un dimère d'oligonucléotide avant comprenant :
(i) un oligonucléotide de ligation avant pour une ligation à l'adaptateur pré-attaché en 5' de la matrice d'ADNc simple brin post-association ; et
(ii) un oligonucléotide de liaison avant pour s'hybrider à l'adaptateur pré-attaché en 5' et à l'oligonucléotide de ligation avant, l'oligonucléotide de liaison avant comprenant une région
complémentaire à l'adaptateur pré-attaché en 5' et une région complémentaire à l'oligonucléotide de ligation avant,
de telle sorte que, lors de l'hybridation, une extrémité de l'oligonucléotide de ligation avant soit adjacente à une extrémité de l'adaptateur pré-attaché en 5' pour permettre une ligation de l'oligonucléotide de ligation avant à l'adaptateur pré-attaché en 5' au niveau d'un site de ligation ; et
(B) un dimère d'oligonucléotide arrière comprenant :
(i) un oligonucléotide de ligation arrière pour une ligation à l'adaptateur pré-attaché en 3' de la matrice d'ADNc simple brin post-association ; et
(ii) un oligonucléotide de liaison arrière pour s'hybrider à l'adaptateur pré-attaché en 3' et à l'oligonucléotide de ligation arrière, l'oligonucléotide de liaison arrière comprenant une région complémentaire à l'adaptateur pré-attaché en 3' et une région complémentaire à l'oligonucléotide de ligation arrière,
de telle sorte que, lors de l'hybridation, une extrémité de l'oligonucléotide de ligation arrière soit adjacente à une extrémité de l'adaptateur pré-attaché en 3' pour permettre une ligation de l'oligonucléotide de ligation arrière à l'adaptateur pré-attaché en 3' au niveau d'un site de ligation.
